# EUROPEAN PATENT APPLICATION

(11) **EP 4 071 246 A1**
(43) Date of publication of application: **12.10.2022**
(21) Application number: 19955387.6
(22) Date of filing: 02.12.2019
(51) Int. Cl.: C12N 11/091

(54) **CO-IMMOBILIZED ENZYME, PREPARATION METHOD THEREFOR AND APPLICATION THEREOF**

(71) Applicant: Jilin Asymchem Laboratories Co., Ltd., Dunhua, Jilin 133700 (CN)
(72) Inventor: HONG, Hao, Morrisville NC 27560 (US); JAMES, Gage, Morrisville NC 27560 (US); RAJASEKAR, Vyasarayani Williams, Tianjin 300457 (CN); CUI, Yuxia, Tianjin 300457 (CN); ZHANG, Na, Tianjin 300457 (CN); ZHAO, Jiadong, Tianjin 300457 (CN); HAO, Mingmin, Tianjin 300457 (CN); GAO, Yanyan, Tianjin 300457 (CN)
(74) Representative: Glawe, Delfs, Moll
(86) International application number: PCT/CN2019/122447
(87) International publication number: WO 2021/108957

(57) **Abstract**

Provided are a co-immobilized enzyme, a preparation method and use thereof. The co-immobilized enzyme includes: an amino resin carrier, a main enzyme, and a coenzyme. The main enzyme and the coenzyme are co-immobilized on the amino resin carrier, herein the main enzyme is covalent-immobilized on the amino resin carrier, and the coenzyme is immobilized on the amino resin carrier by a mode of covalent and/or non-covalent; and the main enzyme is selected from any one of the following enzymes: transaminase, amino acid dehydrogenase, imine reductase, ketoreductase, enoyl reductase, and monooxygenase. The main enzyme and the coenzyme thereof are co-immobilized on the amino resin carrier for co-immobilization, so the activity and the recycling efficiency of the enzyme are improved.

SEQUENCE LISTING

<110>  JILIN ASYMCHEM LABORATORIES CO.,LTD

<120>  Immobilized Enzyme,Preparation method and Use thereof

<130>  PN110371KLY

<160>  9

<170>  PatentIn version 3.5

<210>  1
<211>  253
<212>  PRT
<213>  Ketoreductase(Acetobacter sp. )

<220>
<221>  VARIANT
<222>  (1)..(253)
<223>  KRED-Ac-V2

<220>
<221>  MUTAGEN
<222>  (1)..(253)
<223>  E144S+A94T+N156T

<400>  1

Met Ala Arg Val Ala Gly Lys Val Ala Ile Val Ser Gly Ala Ala Asn
1               5                   10                  15

Gly Ile Gly Lys Ala Thr Ala Gln Leu Leu Ala Lys Glu Gly Ala Lys
            20                  25                  30

Val Val Ile Gly Asp Leu Lys Glu Glu Asp Gly Gln Lys Ala Val Ala
        35                  40                  45

Glu Ile Lys Ala Ala Gly Gly Glu Ala Ala Phe Val Lys Leu Asn Val
    50                  55                  60

Thr Asp Glu Ala Ala Trp Lys Ala Ala Ile Gly Gln Thr Leu Lys Leu
65                  70                  75                  80

Tyr Gly Arg Leu Asp Ile Ala Val Asn Asn Ala Gly Ile Thr Tyr Ser
                85                  90                  95

Gly Ser Val Glu Ser Thr Ser Leu Glu Asp Trp Arg Arg Val Gln Ser
            100                 105                 110

Ile Asn Leu Asp Gly Val Phe Leu Gly Thr Gln Val Ala Ile Glu Ala

        115                 120                 125

Met Lys Lys Ser Gly Gly Gly Ser Ile Val Asn Leu Ser Ser Ile Ser

130                 135                 140

Gly Leu Ile Gly Asp Pro Met Leu Ala Ala Tyr Thr Ala Ser Lys Gly
145                 150                 155                 160

Gly Val Arg Leu Phe Thr Lys Ser Ala Ala Leu His Cys Ala Lys Ser
                165                 170                 175

Gly Tyr Lys Ile Arg Val Asn Ser Val His Pro Gly Tyr Ile Trp Thr
            180                 185                 190

Pro Met Val Ala Gly Leu Thr Lys Glu Asp Ala Ala Ala Arg Gln Lys
        195                 200                 205

Leu Val Asp Leu His Pro Ile Gly His Leu Gly Glu Pro Asn Asp Ile
    210                 215                 220

Ala Tyr Gly Ile Leu Tyr Leu Ala Ser Asp Glu Ser Lys Phe Val Thr
225                 230                 235                 240

Gly Ser Glu Leu Val Ile Asp Gly Gly Tyr Thr Ala Gln
                245                 250

<210>  2
<211>  253
<212>  PRT
<213>  Ketoreductase(Acetobacter sp. )

<220>
<221>  VARIANT
<222>  (1)..(253)
<223>  KRED-Ac-V1

<220>
<221>  MUTAGEN
<222>  (1)..(253)
<223>  E144S+A94N+N156V

<400>  2

Met Ala Arg Val Ala Gly Lys Val Ala Ile Val Ser Gly Ala Ala Asn
1               5                   10                  15

Gly Ile Gly Lys Ala Thr Ala Gln Leu Leu Ala Lys Glu Gly Ala Lys
            20                  25                  30

Val Val Ile Gly Asp Leu Lys Glu Glu Asp Gly Gln Lys Ala Val Ala
        35                  40                  45

Glu Ile Lys Ala Ala Gly Gly Glu Ala Ala Phe Val Lys Leu Asn Val
    50                  55                  60

Thr Asp Glu Ala Ala Trp Lys Ala Ala Ile Gly Gln Thr Leu Lys Leu
65                  70                  75                  80

Tyr Gly Arg Leu Asp Ile Ala Val Asn Asn Ala Gly Ile Asn Tyr Ser
                85                  90                  95

Gly Ser Val Glu Ser Thr Ser Leu Glu Asp Trp Arg Arg Val Gln Ser
            100                 105                 110

Ile Asn Leu Asp Gly Val Phe Leu Gly Thr Gln Val Ala Ile Glu Ala
        115                 120                 125

Met Lys Lys Ser Gly Gly Gly Ser Ile Val Asn Leu Ser Ser Ile Ser
    130                 135                 140

Gly Leu Ile Gly Asp Pro Met Leu Ala Ala Tyr Val Ala Ser Lys Gly
145                 150                 155                 160

Gly Val Arg Leu Phe Thr Lys Ser Ala Ala Leu His Cys Ala Lys Ser
                165                 170                 175

Gly Tyr Lys Ile Arg Val Asn Ser Val His Pro Gly Tyr Ile Trp Thr
            180                 185                 190

Pro Met Val Ala Gly Leu Thr Lys Glu Asp Ala Ala Ala Arg Gln Lys
        195                 200                 205

Leu Val Asp Leu His Pro Ile Gly His Leu Gly Glu Pro Asn Asp Ile
    210                 215                 220

Ala Tyr Gly Ile Leu Tyr Leu Ala Ser Asp Glu Ser Lys Phe Val Thr
225                 230                 235                 240

Gly Ser Glu Leu Val Ile Asp Gly Gly Tyr Thr Ala Gln
                245                 250

<210>  3
<211>  253
<212>  PRT
<213>  Ketoreductase(Acetobacter sp. )

<220>
<221>  SITE
<222>  (1)..(253)
<223>  KRED-Ac-mather line

<400>  3

Met Ala Arg Val Ala Gly Lys Val Ala Ile Val Ser Gly Ala Ala Asn
1               5                   10                  15

Gly Ile Gly Lys Ala Thr Ala Gln Leu Leu Ala Lys Glu Gly Ala Lys
            20                  25                  30

Val Val Ile Gly Asp Leu Lys Glu Glu Asp Gly Gln Lys Ala Val Ala
        35                  40                  45

Glu Ile Lys Ala Ala Gly Gly Glu Ala Ala Phe Val Lys Leu Asn Val
    50                  55                  60

Thr Asp Glu Ala Ala Trp Lys Ala Ala Ile Gly Gln Thr Leu Lys Leu
65                  70                  75                  80

Tyr Gly Arg Leu Asp Ile Ala Val Asn Asn Ala Gly Ile Ala Tyr Ser
                85                  90                  95

Gly Ser Val Glu Ser Thr Ser Leu Glu Asp Trp Arg Arg Val Gln Ser
            100                 105                 110

Ile Asn Leu Asp Gly Val Phe Leu Gly Thr Gln Val Ala Ile Glu Ala
        115                 120                 125

Met Lys Lys Ser Gly Gly Gly Ser Ile Val Asn Leu Ser Ser Ile Glu
    130                 135                 140

Gly Leu Ile Gly Asp Pro Met Leu Ala Ala Tyr Asn Ala Ser Lys Gly
145                 150                 155                 160

Gly Val Arg Leu Phe Thr Lys Ser Ala Ala Leu His Cys Ala Lys Ser
                165                 170                 175

Gly Tyr Lys Ile Arg Val Asn Ser Val His Pro Gly Tyr Ile Trp Thr
            180                 185                 190

Pro Met Val Ala Gly Leu Thr Lys Glu Asp Ala Ala Ala Arg Gln Lys
        195                 200                 205

Leu Val Asp Leu His Pro Ile Gly His Leu Gly Glu Pro Asn Asp Ile
    210                 215                 220

Ala Tyr Gly Ile Leu Tyr Leu Ala Ser Asp Glu Ser Lys Phe Val Thr
225                 230                 235                 240

Gly Ser Glu Leu Val Ile Asp Gly Gly Tyr Thr Ala Gln
                245                 250

<210>  4
<211>  541
<212>  PRT
<213>  Cyclohexanone monooxygenase(Rhodococcus sp. Phi1)

<220>
<221>  VARIANT
<222>  (1)..(541)
<223>  CHMO-Rs-V2

<220>
<221>  MUTAGEN
<222>  (1)..(541)
<223>  F508Y+F435N+L438A+T436S+F280V+S441V+L510V

<400>  4

Met Thr Ala Gln Ile Ser Pro Thr Val Val Asp Ala Val Val Ile Gly
1               5                   10                  15

Ala Gly Phe Gly Gly Ile Tyr Ala Val His Lys Leu His Asn Glu Gln
            20                  25                  30

Gly Leu Thr Val Val Gly Phe Asp Lys Ala Asp Gly Pro Gly Gly Thr
        35                  40                  45

Trp Tyr Trp Asn Arg Tyr Pro Gly Ala Leu Ser Asp Thr Glu Ser His
    50                  55                  60

Leu Tyr Arg Phe Ser Phe Asp Arg Asp Leu Leu Gln Asp Gly Thr Trp
65                  70                  75                  80

Lys Thr Thr Tyr Ile Thr Gln Pro Glu Ile Leu Glu Tyr Leu Glu Ser
                85                  90                  95

Val Val Asp Arg Phe Asp Leu Arg Arg His Phe Arg Phe Gly Thr Glu
            100                 105                 110

Val Thr Ser Ala Ile Tyr Leu Glu Asp Glu Asn Leu Trp Glu Val Ser
        115                 120                 125

Thr Asp Lys Gly Glu Val Tyr Arg Ala Lys Tyr Val Val Asn Ala Val
    130                 135                 140

Gly Leu Leu Ser Ala Ile Asn Phe Pro Asp Leu Pro Gly Leu Asp Thr
145                 150                 155                 160

Phe Glu Gly Glu Thr Ile His Thr Ala Ala Trp Pro Glu Gly Lys Asn
                165                 170                 175

Leu Ala Gly Lys Arg Val Gly Val Ile Gly Thr Gly Ser Thr Gly Gln
        180                 185                 190

Gln Val Ile Thr Ala Leu Ala Pro Glu Val Glu His Leu Thr Val Phe
    195                 200                 205

Val Arg Thr Pro Gln Tyr Ser Val Pro Val Gly Asn Arg Pro Val Thr
    210                 215                 220

Lys Glu Gln Ile Asp Ala Ile Lys Ala Asp Tyr Asp Gly Ile Trp Asp
225                 230                 235                 240

Ser Val Lys Lys Ser Ala Val Ala Phe Gly Phe Glu Glu Ser Thr Leu
                245                 250                 255

Pro Ala Met Ser Val Ser Glu Glu Glu Arg Asn Arg Ile Phe Gln Glu
            260                 265                 270

Ala Trp Asp His Gly Gly Gly Val Arg Phe Met Phe Gly Thr Phe Gly
        275                 280                 285

Asp Ile Ala Thr Asp Glu Ala Ala Asn Glu Ala Ala Ala Ser Phe Ile
    290                 295                 300

Arg Ser Lys Ile Ala Glu Ile Ile Glu Asp Pro Glu Thr Ala Arg Lys
305                 310                 315                 320

Leu Met Pro Thr Gly Leu Tyr Ala Lys Arg Pro Leu Cys Asp Asn Gly
                325                 330                 335

Tyr Tyr Glu Val Tyr Asn Arg Pro Asn Val Glu Ala Val Ala Ile Lys
            340                 345                 350

Glu Asn Pro Ile Arg Glu Val Thr Ala Lys Gly Val Val Thr Glu Asp
        355                 360                 365

Gly Val Leu His Glu Leu Asp Val Leu Val Phe Ala Thr Gly Phe Asp
    370                 375                 380

Ala Val Asp Gly Asn Tyr Arg Arg Ile Glu Ile Arg Gly Arg Asn Gly
385                 390                 395                 400

Leu His Ile Asn Asp His Trp Asp Gly Gln Pro Thr Ser Tyr Leu Gly
                405                 410                 415

Val Thr Thr Ala Asn Phe Pro Asn Trp Phe Met Val Leu Gly Pro Asn
            420                 425                 430

Gly Pro Asn Ser Asn Ala Pro Pro Val Ile Glu Thr Gln Val Glu Trp
        435                 440                 445

Ile Ser Asp Thr Val Ala Tyr Ala Glu Arg Asn Glu Ile Arg Ala Ile
    450                 455                 460

Glu Pro Thr Pro Glu Ala Glu Glu Glu Trp Thr Gln Thr Cys Thr Asp
465                 470                 475                 480

Ile Ala Asn Ala Thr Leu Phe Thr Arg Gly Asp Ser Trp Ile Phe Gly
                485                 490                 495

Ala Asn Val Pro Gly Lys Lys Pro Ser Val Leu Tyr Tyr Val Gly Gly
            500                 505                 510

Leu Gly Asn Tyr Arg Asn Val Leu Ala Gly Val Val Ala Asp Ser Tyr
        515                 520                 525

Arg Gly Phe Glu Leu Lys Ser Ala Val Pro Val Thr Ala
    530                 535                 540

<210>  5
<211>  541
<212>  PRT
<213>  Cyclohexanone monooxygenase(Rhodococcus sp. Phi1)

<220>
<221>  VARIANT
<222>  (1)..(541)
<223>  CHMO-Rs-V1

<220>
<221>  MUTAGEN
<222>  (1)..(541)
<223>  F508Y+F435N+L438A+T436S+F280V+S441V

<400>  5

Met Thr Ala Gln Ile Ser Pro Thr Val Val Asp Ala Val Val Ile Gly
1               5                   10                  15

Ala Gly Phe Gly Gly Ile Tyr Ala Val His Lys Leu His Asn Glu Gln
            20                  25                  30

Gly Leu Thr Val Val Gly Phe Asp Lys Ala Asp Gly Pro Gly Gly Thr
        35                  40                  45

Trp Tyr Trp Asn Arg Tyr Pro Gly Ala Leu Ser Asp Thr Glu Ser His
    50                  55                  60

Leu Tyr Arg Phe Ser Phe Asp Arg Asp Leu Leu Gln Asp Gly Thr Trp
65                  70                  75                  80

Lys Thr Thr Tyr Ile Thr Gln Pro Glu Ile Leu Glu Tyr Leu Glu Ser
                85                  90                  95

Val Val Asp Arg Phe Asp Leu Arg Arg His Phe Arg Phe Gly Thr Glu
            100                 105                 110

Val Thr Ser Ala Ile Tyr Leu Glu Asp Glu Asn Leu Trp Glu Val Ser
        115                 120                 125

Thr Asp Lys Gly Glu Val Tyr Arg Ala Lys Tyr Val Val Asn Ala Val
    130                 135                 140

Gly Leu Leu Ser Ala Ile Asn Phe Pro Asp Leu Pro Gly Leu Asp Thr
145                 150                 155                 160

Phe Glu Gly Glu Thr Ile His Thr Ala Ala Trp Pro Glu Gly Lys Asn
                165                 170                 175

Leu Ala Gly Lys Arg Val Gly Val Ile Gly Thr Gly Ser Thr Gly Gln
            180                 185                 190

Gln Val Ile Thr Ala Leu Ala Pro Glu Val Glu His Leu Thr Val Phe
        195                 200                 205

Val Arg Thr Pro Gln Tyr Ser Val Pro Val Gly Asn Arg Pro Val Thr
    210                 215                 220

Lys Glu Gln Ile Asp Ala Ile Lys Ala Asp Tyr Asp Gly Ile Trp Asp
225                 230                 235                 240

Ser Val Lys Lys Ser Ala Val Ala Phe Gly Phe Glu Glu Ser Thr Leu
                245                 250                 255

Pro Ala Met Ser Val Ser Glu Glu Glu Arg Asn Arg Ile Phe Gln Glu
            260                 265                 270

Ala Trp Asp His Gly Gly Gly Val Arg Phe Met Phe Gly Thr Phe Gly
        275                 280                 285

Asp Ile Ala Thr Asp Glu Ala Ala Asn Glu Ala Ala Ala Ser Phe Ile
    290                 295                 300

Arg Ser Lys Ile Ala Glu Ile Ile Glu Asp Pro Glu Thr Ala Arg Lys
305                 310                 315                 320

Leu Met Pro Thr Gly Leu Tyr Ala Lys Arg Pro Leu Cys Asp Asn Gly
                325                 330                 335

Tyr Tyr Glu Val Tyr Asn Arg Pro Asn Val Glu Ala Val Ala Ile Lys
            340                 345                 350

Glu Asn Pro Ile Arg Glu Val Thr Ala Lys Gly Val Val Thr Glu Asp
        355                 360                 365

Gly Val Leu His Glu Leu Asp Val Leu Val Phe Ala Thr Gly Phe Asp
    370                 375                 380

Ala Val Asp Gly Asn Tyr Arg Arg Ile Glu Ile Arg Gly Arg Asn Gly
385                 390                 395                 400

Leu His Ile Asn Asp His Trp Asp Gly Gln Pro Thr Ser Tyr Leu Gly
                405                 410                 415

Val Thr Thr Ala Asn Phe Pro Asn Trp Phe Met Val Leu Gly Pro Asn
            420                 425                 430

Gly Pro Asn Ser Asn Ala Pro Pro Val Ile Glu Thr Gln Val Glu Trp
        435                 440                 445

Ile Ser Asp Thr Val Ala Tyr Ala Glu Arg Asn Glu Ile Arg Ala Ile
    450                 455                 460

Glu Pro Thr Pro Glu Ala Glu Glu Glu Trp Thr Gln Thr Cys Thr Asp
465                 470                 475                 480

Ile Ala Asn Ala Thr Leu Phe Thr Arg Gly Asp Ser Trp Ile Phe Gly
                485                 490                 495

Ala Asn Val Pro Gly Lys Lys Pro Ser Val Leu Tyr Tyr Leu Gly Gly
            500                 505                 510

Leu Gly Asn Tyr Arg Asn Val Leu Ala Gly Val Val Ala Asp Ser Tyr
        515                 520                 525

Arg Gly Phe Glu Leu Lys Ser Ala Val Pro Val Thr Ala
    530                 535                 540

<210>  6
<211>  541
<212>  PRT
<213>  Cyclohexanone monooxygenase(Rhodococcus sp. Phi1)

<220>
<221>  SITE
<222>  (1)..(541)
<223>  CHMO-Rs-mather line

<400>  6

Met Thr Ala Gln Ile Ser Pro Thr Val Val Asp Ala Val Val Ile Gly
1               5                   10                  15

Ala Gly Phe Gly Gly Ile Tyr Ala Val His Lys Leu His Asn Glu Gln
            20                  25                  30

Gly Leu Thr Val Val Gly Phe Asp Lys Ala Asp Gly Pro Gly Gly Thr
        35                  40                  45

Trp Tyr Trp Asn Arg Tyr Pro Gly Ala Leu Ser Asp Thr Glu Ser His
    50                  55                  60

Leu Tyr Arg Phe Ser Phe Asp Arg Asp Leu Leu Gln Asp Gly Thr Trp
65                  70                  75                  80

Lys Thr Thr Tyr Ile Thr Gln Pro Glu Ile Leu Glu Tyr Leu Glu Ser
                85                  90                  95

Val Val Asp Arg Phe Asp Leu Arg Arg His Phe Arg Phe Gly Thr Glu
            100                 105                 110

Val Thr Ser Ala Ile Tyr Leu Glu Asp Glu Asn Leu Trp Glu Val Ser
        115                 120                 125

Thr Asp Lys Gly Glu Val Tyr Arg Ala Lys Tyr Val Val Asn Ala Val
    130                 135                 140

Gly Leu Leu Ser Ala Ile Asn Phe Pro Asp Leu Pro Gly Leu Asp Thr
145                 150                 155                 160

Phe Glu Gly Glu Thr Ile His Thr Ala Ala Trp Pro Glu Gly Lys Asn
                165                 170                 175

Leu Ala Gly Lys Arg Val Gly Val Ile Gly Thr Gly Ser Thr Gly Gln
            180                 185                 190

Gln Val Ile Thr Ala Leu Ala Pro Glu Val Glu His Leu Thr Val Phe
        195                 200                 205

Val Arg Thr Pro Gln Tyr Ser Val Pro Val Gly Asn Arg Pro Val Thr
    210                 215                 220

Lys Glu Gln Ile Asp Ala Ile Lys Ala Asp Tyr Asp Gly Ile Trp Asp
225                 230                 235                 240

Ser Val Lys Lys Ser Ala Val Ala Phe Gly Phe Glu Glu Ser Thr Leu
                245                 250                 255

Pro Ala Met Ser Val Ser Glu Glu Glu Arg Asn Arg Ile Phe Gln Glu
            260                 265                 270

Ala Trp Asp His Gly Gly Gly Phe Arg Phe Met Phe Gly Thr Phe Gly
        275                 280                 285

Asp Ile Ala Thr Asp Glu Ala Ala Asn Glu Ala Ala Ala Ser Phe Ile
    290                 295                 300

Arg Ser Lys Ile Ala Glu Ile Ile Glu Asp Pro Glu Thr Ala Arg Lys
305                 310                 315                 320

Leu Met Pro Thr Gly Leu Tyr Ala Lys Arg Pro Leu Cys Asp Asn Gly
                325                 330                 335

Tyr Tyr Glu Val Tyr Asn Arg Pro Asn Val Glu Ala Val Ala Ile Lys
            340                 345                 350

Glu Asn Pro Ile Arg Glu Val Thr Ala Lys Gly Val Val Thr Glu Asp
        355                 360                 365

Gly Val Leu His Glu Leu Asp Val Leu Val Phe Ala Thr Gly Phe Asp
    370                 375                 380

Ala Val Asp Gly Asn Tyr Arg Arg Ile Glu Ile Arg Gly Arg Asn Gly
385                 390                 395                 400

Leu His Ile Asn Asp His Trp Asp Gly Gln Pro Thr Ser Tyr Leu Gly
                405                 410                 415

Val Thr Thr Ala Asn Phe Pro Asn Trp Phe Met Val Leu Gly Pro Asn
            420                 425                 430

Gly Pro Phe Thr Asn Leu Pro Pro Ser Ile Glu Thr Gln Val Glu Trp
        435                 440                 445

Ile Ser Asp Thr Val Ala Tyr Ala Glu Arg Asn Glu Ile Arg Ala Ile
    450                 455                 460

Glu Pro Thr Pro Glu Ala Glu Glu Glu Trp Thr Gln Thr Cys Thr Asp
465                 470                 475                 480

Ile Ala Asn Ala Thr Leu Phe Thr Arg Gly Asp Ser Trp Ile Phe Gly
                485                 490                 495

Ala Asn Val Pro Gly Lys Lys Pro Ser Val Leu Phe Tyr Leu Gly Gly
            500                 505                 510

Leu Gly Asn Tyr Arg Asn Val Leu Ala Gly Val Val Ala Asp Ser Tyr
        515                 520                 525

Arg Gly Phe Glu Leu Lys Ser Ala Val Pro Val Thr Ala
    530                 535                 540

<210>  7
<211>  603
<212>  PRT
<213>  Cyclohexanone monooxygenase(Rhodococcus ruber-SD1)

<220>
<221>  VARIANT
<222>  (1)..(603)
<223>  CHMO-Rr-V2

<220>
<221>  MUTAGEN
<222>  (1)..(603)
<223>  Y559M+P190L+P504V

<400>  7

Met Thr Thr Ser Ile Asp Arg Glu Ala Leu Arg Arg Lys Tyr Ala Glu
1               5                   10                  15

Glu Arg Asp Lys Arg Ile Arg Pro Asp Gly Asn Asp Gln Tyr Ile Arg
            20                  25                  30

Leu Asp His Val Asp Gly Trp Ser His Asp Pro Tyr Met Pro Ile Thr
        35                  40                  45

Pro Arg Glu Pro Lys Leu Asp His Val Thr Phe Ala Phe Ile Gly Gly
    50                  55                  60

Gly Phe Ser Gly Leu Val Thr Ala Ala Arg Leu Arg Glu Ser Gly Val
65                  70                  75                  80

Glu Ser Val Arg Ile Ile Asp Lys Ala Gly Asp Phe Gly Gly Val Trp
                85                  90                  95

Tyr Trp Asn Arg Tyr Pro Gly Ala Met Cys Asp Thr Ala Ala Met Val
            100                 105                 110

Tyr Met Pro Leu Leu Glu Glu Thr Gly Tyr Met Pro Thr Glu Lys Tyr
        115                 120                 125

Ala His Gly Pro Glu Ile Leu Glu His Cys Gln Arg Ile Gly Lys His
    130                 135                 140

Tyr Asp Leu Tyr Asp Asp Ala Leu Phe His Thr Glu Val Thr Asp Leu
145                 150                 155                 160

Val Trp Gln Glu His Asp Gln Arg Trp Arg Ile Ser Thr Asn Arg Gly
                165                 170                 175

Asp His Phe Thr Ala Gln Phe Val Gly Met Gly Thr Gly Leu Leu His
            180                 185                 190

Val Ala Gln Leu Pro Gly Ile Pro Gly Ile Glu Ser Phe Arg Gly Lys
        195                 200                 205

Ser Phe His Thr Ser Arg Trp Asp Tyr Asp Tyr Thr Gly Gly Asp Ala
    210                 215                 220

Leu Gly Ala Pro Met Asp Lys Leu Ala Asp Lys Arg Val Ala Val Ile
225                 230                 235                 240

Gly Thr Gly Ala Thr Ala Val Gln Cys Val Pro Glu Leu Ala Lys Tyr
                245                 250                 255

Cys Arg Glu Leu Tyr Val Val Gln Arg Thr Pro Ser Ala Val Asp Glu
            260                 265                 270

Arg Gly Asn His Pro Ile Asp Glu Lys Trp Phe Ala Gln Ile Ala Thr
        275                 280                 285

Pro Gly Trp Gln Lys Arg Trp Leu Asp Ser Phe Thr Ala Ile Trp Asp
    290                 295                 300

Gly Val Leu Thr Asp Pro Ser Glu Leu Ala Ile Glu His Glu Asp Leu
305                 310                 315                 320

Val Gln Asp Gly Trp Thr Ala Leu Gly Gln Arg Met Arg Ala Ala Val
                325                 330                 335

Gly Ser Val Pro Ile Glu Gln Tyr Ser Pro Glu Asn Val Gln Arg Ala
            340                 345                 350

Leu Glu Glu Ala Asp Asp Glu Gln Met Glu Arg Ile Arg Ala Arg Val
        355                 360                 365

Asp Glu Ile Val Thr Asp Pro Ala Thr Ala Ala Gln Leu Lys Ala Trp
    370                 375                 380

Phe Arg Gln Met Cys Lys Arg Pro Cys Phe His Asp Asp Tyr Leu Pro
385                 390                 395                 400

Ala Phe Asn Arg Pro Asn Thr His Leu Val Asp Thr Gly Gly Lys Gly
                405                 410                 415

Val Glu Arg Ile Thr Glu Asn Gly Val Val Val Ala Gly Val Glu Tyr
            420                 425                 430

Glu Val Asp Cys Ile Val Tyr Ala Ser Gly Phe Glu Phe Leu Gly Thr
        435                 440                 445

Gly Tyr Thr Asp Arg Ala Gly Phe Asp Pro Thr Gly Arg Asp Gly Val
    450                 455                 460

Lys Leu Ser Glu His Trp Ala Gln Gly Thr Arg Thr Leu His Gly Met
465                 470                 475                 480

His Thr Tyr Gly Phe Pro Asn Leu Phe Val Leu Gln Leu Met Gln Gly
                485                 490                 495

Ala Ala Leu Gly Ser Asn Ile Val His Asn Phe Val Glu Ala Ala Arg
            500                 505                 510

Val Val Ala Ala Ile Val Asp His Val Leu Ser Thr Gly Thr Ser Ser
        515                 520                 525

Val Glu Thr Thr Lys Glu Ala Glu Gln Ala Trp Val Gln Leu Leu Leu
    530                 535                 540

Asp His Gly Arg Pro Leu Gly Asn Pro Glu Cys Thr Pro Gly Met Tyr
545                 550                 555                 560

Asn Asn Glu Gly Lys Pro Ala Glu Leu Lys Asp Arg Leu Asn Val Gly
                565                 570                 575

Tyr Pro Ala Gly Ser Ala Ala Phe Phe Arg Met Met Asp His Trp Leu
            580                 585                 590

Ala Ala Gly Ser Phe Asp Gly Leu Thr Phe Arg
        595                 600

<210>  8
<211>  603

<212>  PRT
<213>  Cyclohexanone monooxygenase(Rhodococcus ruber-SD1)

<220>
<221>  VARIANT
<222>  (1)..(603)
<223>  CHMO-Rr-V1

<220>
<221>  MUTAGEN
<222>  (1)..(603)
<223>  P190L+Y559M+C249V+C393V+C257A+M45T

<400>  8

Met Thr Thr Ser Ile Asp Arg Glu Ala Leu Arg Arg Lys Tyr Ala Glu
1               5                   10                  15

Glu Arg Asp Lys Arg Ile Arg Pro Asp Gly Asn Asp Gln Tyr Ile Arg
            20                  25                  30

Leu Asp His Val Asp Gly Trp Ser His Asp Pro Tyr Thr Pro Ile Thr
        35                  40                  45

Pro Arg Glu Pro Lys Leu Asp His Val Thr Phe Ala Phe Ile Gly Gly
    50                  55                  60

Gly Phe Ser Gly Leu Val Thr Ala Ala Arg Leu Arg Glu Ser Gly Val
65                  70                  75                  80

Glu Ser Val Arg Ile Ile Asp Lys Ala Gly Asp Phe Gly Gly Val Trp
                85                  90                  95

Tyr Trp Asn Arg Tyr Pro Gly Ala Met Cys Asp Thr Ala Ala Met Val
            100                 105                 110

Tyr Met Pro Leu Leu Glu Glu Thr Gly Tyr Met Pro Thr Glu Lys Tyr
        115                 120                 125

Ala His Gly Pro Glu Ile Leu Glu His Cys Gln Arg Ile Gly Lys His
    130                 135                 140

Tyr Asp Leu Tyr Asp Asp Ala Leu Phe His Thr Glu Val Thr Asp Leu
145                 150                 155                 160

Val Trp Gln Glu His Asp Gln Arg Trp Arg Ile Ser Thr Asn Arg Gly
                165                 170                 175

Asp His Phe Thr Ala Gln Phe Val Gly Met Gly Thr Gly Leu Leu His
            180                 185                 190

Val Ala Gln Leu Pro Gly Ile Pro Gly Ile Glu Ser Phe Arg Gly Lys
        195                 200                 205

Ser Phe His Thr Ser Arg Trp Asp Tyr Asp Tyr Thr Gly Gly Asp Ala
    210                 215                 220

Leu Gly Ala Pro Met Asp Lys Leu Ala Asp Lys Arg Val Ala Val Ile
225                 230                 235                 240

Gly Thr Gly Ala Thr Ala Val Gln Val Val Pro Glu Leu Ala Lys Tyr
                245                 250                 255

Ala Arg Glu Leu Tyr Val Val Gln Arg Thr Pro Ser Ala Val Asp Glu
            260                 265                 270

Arg Gly Asn His Pro Ile Asp Glu Lys Trp Phe Ala Gln Ile Ala Thr
        275                 280                 285

Pro Gly Trp Gln Lys Arg Trp Leu Asp Ser Phe Thr Ala Ile Trp Asp
    290                 295                 300

Gly Val Leu Thr Asp Pro Ser Glu Leu Ala Ile Glu His Glu Asp Leu
305                 310                 315                 320

Val Gln Asp Gly Trp Thr Ala Leu Gly Gln Arg Met Arg Ala Ala Val
                325                 330                 335

Gly Ser Val Pro Ile Glu Gln Tyr Ser Pro Glu Asn Val Gln Arg Ala
            340                 345                 350

Leu Glu Glu Ala Asp Asp Glu Gln Met Glu Arg Ile Arg Ala Arg Val
        355                 360                 365

Asp Glu Ile Val Thr Asp Pro Ala Thr Ala Ala Gln Leu Lys Ala Trp
    370                 375                 380

Phe Arg Gln Met Cys Lys Arg Pro Val Phe His Asp Asp Tyr Leu Pro
385                 390                 395                 400

Ala Phe Asn Arg Pro Asn Thr His Leu Val Asp Thr Gly Gly Lys Gly
                405                 410                 415

Val Glu Arg Ile Thr Glu Asn Gly Val Val Val Ala Gly Val Glu Tyr
            420                 425                 430

Glu Val Asp Cys Ile Val Tyr Ala Ser Gly Phe Glu Phe Leu Gly Thr
        435                 440                 445

Gly Tyr Thr Asp Arg Ala Gly Phe Asp Pro Thr Gly Arg Asp Gly Val
    450                 455                 460

Lys Leu Ser Glu His Trp Ala Gln Gly Thr Arg Thr Leu His Gly Met
465                 470                 475                 480

His Thr Tyr Gly Phe Pro Asn Leu Phe Val Leu Gln Leu Met Gln Gly
                485                 490                 495

Ala Ala Leu Gly Ser Asn Ile Pro His Asn Phe Val Glu Ala Ala Arg
            500                 505                 510

Val Val Ala Ala Ile Val Asp His Val Leu Ser Thr Gly Thr Ser Ser
        515                 520                 525

Val Glu Thr Thr Lys Glu Ala Glu Gln Ala Trp Val Gln Leu Leu Leu
    530                 535                 540

Asp His Gly Arg Pro Leu Gly Asn Pro Glu Cys Thr Pro Gly Met Tyr
545                 550                 555                 560

Asn Asn Glu Gly Lys Pro Ala Glu Leu Lys Asp Arg Leu Asn Val Gly
                565                 570                 575

Tyr Pro Ala Gly Ser Ala Ala Phe Phe Arg Met Met Asp His Trp Leu
            580                 585                 590

Ala Ala Gly Ser Phe Asp Gly Leu Thr Phe Arg
        595                 600

<210>  9
<211>  603
<212>  PRT
<213>  Cyclohexanone monooxygenase(Rhodococcus ruber-SD1)

<220>
<221>  SITE
<222>  (1)..(603)
<223>  CHMO-Rr-mather line

<400>  9

Met Thr Thr Ser Ile Asp Arg Glu Ala Leu Arg Arg Lys Tyr Ala Glu
1               5                   10                  15

Glu Arg Asp Lys Arg Ile Arg Pro Asp Gly Asn Asp Gln Tyr Ile Arg
            20                  25                  30

Leu Asp His Val Asp Gly Trp Ser His Asp Pro Tyr Met Pro Ile Thr
        35                  40                  45

Pro Arg Glu Pro Lys Leu Asp His Val Thr Phe Ala Phe Ile Gly Gly
    50                  55                  60

Gly Phe Ser Gly Leu Val Thr Ala Ala Arg Leu Arg Glu Ser Gly Val
65                  70                  75                  80

Glu Ser Val Arg Ile Ile Asp Lys Ala Gly Asp Phe Gly Gly Val Trp
                85                  90                  95

Tyr Trp Asn Arg Tyr Pro Gly Ala Met Cys Asp Thr Ala Ala Met Val
            100                 105                 110

Tyr Met Pro Leu Leu Glu Glu Thr Gly Tyr Met Pro Thr Glu Lys Tyr
        115                 120                 125

Ala His Gly Pro Glu Ile Leu Glu His Cys Gln Arg Ile Gly Lys His
    130                 135                 140

Tyr Asp Leu Tyr Asp Asp Ala Leu Phe His Thr Glu Val Thr Asp Leu
145                 150                 155                 160

Val Trp Gln Glu His Asp Gln Arg Trp Arg Ile Ser Thr Asn Arg Gly
                165                 170                 175

Asp His Phe Thr Ala Gln Phe Val Gly Met Gly Thr Gly Pro Leu His
            180                 185                 190

Val Ala Gln Leu Pro Gly Ile Pro Gly Ile Glu Ser Phe Arg Gly Lys
        195                 200                 205

Ser Phe His Thr Ser Arg Trp Asp Tyr Asp Tyr Thr Gly Gly Asp Ala
    210                 215                 220

Leu Gly Ala Pro Met Asp Lys Leu Ala Asp Lys Arg Val Ala Val Ile
225                 230                 235                 240

Gly Thr Gly Ala Thr Ala Val Gln Cys Val Pro Glu Leu Ala Lys Tyr
                245                 250                 255

Cys Arg Glu Leu Tyr Val Val Gln Arg Thr Pro Ser Ala Val Asp Glu
            260                 265                 270

Arg Gly Asn His Pro Ile Asp Glu Lys Trp Phe Ala Gln Ile Ala Thr
        275                 280                 285

Pro Gly Trp Gln Lys Arg Trp Leu Asp Ser Phe Thr Ala Ile Trp Asp
    290                 295                 300

Gly Val Leu Thr Asp Pro Ser Glu Leu Ala Ile Glu His Glu Asp Leu
305                 310                 315                 320

Val Gln Asp Gly Trp Thr Ala Leu Gly Gln Arg Met Arg Ala Ala Val
                325                 330                 335

Gly Ser Val Pro Ile Glu Gln Tyr Ser Pro Glu Asn Val Gln Arg Ala
            340                 345                 350

Leu Glu Glu Ala Asp Asp Glu Gln Met Glu Arg Ile Arg Ala Arg Val
        355                 360                 365

Asp Glu Ile Val Thr Asp Pro Ala Thr Ala Ala Gln Leu Lys Ala Trp
    370                 375                 380

Phe Arg Gln Met Cys Lys Arg Pro Cys Phe His Asp Asp Tyr Leu Pro
385                 390                 395                 400

Ala Phe Asn Arg Pro Asn Thr His Leu Val Asp Thr Gly Gly Lys Gly
                405                 410                 415

Val Glu Arg Ile Thr Glu Asn Gly Val Val Val Ala Gly Val Glu Tyr
            420                 425                 430

Glu Val Asp Cys Ile Val Tyr Ala Ser Gly Phe Glu Phe Leu Gly Thr
        435                 440                 445

Gly Tyr Thr Asp Arg Ala Gly Phe Asp Pro Thr Gly Arg Asp Gly Val
    450                 455                 460

Lys Leu Ser Glu His Trp Ala Gln Gly Thr Arg Thr Leu His Gly Met
465                 470                 475                 480

His Thr Tyr Gly Phe Pro Asn Leu Phe Val Leu Gln Leu Met Gln Gly
                485                 490                 495

Ala Ala Leu Gly Ser Asn Ile Pro His Asn Phe Val Glu Ala Ala Arg
            500                 505                 510

Val Val Ala Ala Ile Val Asp His Val Leu Ser Thr Gly Thr Ser Ser
        515                 520                 525

Val Glu Thr Thr Lys Glu Ala Glu Gln Ala Trp Val Gln Leu Leu Leu
    530                 535                 540

Asp His Gly Arg Pro Leu Gly Asn Pro Glu Cys Thr Pro Gly Tyr Tyr
545                 550                 555                 560

Asn Asn Glu Gly Lys Pro Ala Glu Leu Lys Asp Arg Leu Asn Val Gly
                565                 570                 575

Tyr Pro Ala Gly Ser Ala Ala Phe Phe Arg Met Met Asp His Trp Leu
            580                 585                 590

Ala Ala Gly Ser Phe Asp Gly Leu Thr Phe Arg
        595                 600

## Description

### Technical Field

The present invention relates to the field of immobilized enzymes, in particular to a co-immobilized enzyme, a preparation method therefor and use thereof.

### Background

The applications of microbial cells or separated or engineered enzymes make great progress in biocatalysis, and manufacturing modes are transformed. Many types of enzymes such as an acyltransferase, an amidase, a transaminase, a ketoreductase, an oxidase, a monooxygenase and a hydrolase are used in production of reactions involving antibiotics, herbicides, pharmaceutical intermediates and new age therapeutic agents.

While a free enzyme is used as a biocatalyst, there is a great waste of the enzyme because it is very difficult to recover a water-soluble enzyme. However, a water-insoluble immobilized enzyme may be easily recovered by very simple filtration after each cycle.

Immobilization methods for single enzymes are already reported in existing technologies, but the immobilization methods suitable for the different enzymes are different. For example, Bolivar et al. (Biomacromol. 2006, 7, 669-673) research the covalent immobilization of a formate dehydrogenase (FDH) from pseudomonas SP101, including the covalent immobilization on various carriers such as a modified agarose, a CNBr-activated agarose, Sepabeads (dextran) and an acetaldehyde agarose. It is concluded that the immobilization on activated carriers such as a bromide, a polyethyleneimine, and a glutaraldehyde do not promote any stabilization effects of the enzyme under heat inactivation. However, an optimized enzyme of a highly activated glyoxal agarose is proved to have high heat stability and pH stability, and have more than 50% of the activity in the case of enhanced stability.

Kim et al (J.Mol.Catal B:Enzy 97(2013) 209-214) report that a method of a cross-linked enzyme aggregate (CLEA) is used to immobilize FDH from Candida boidinii, and it is believed that a dextran polyaldehyde as a cross-linking agent instead of a glutaraldehyde is better for immobilizing the enzyme, and the residual activity exceeds 95% after 10 times of repeated uses. In addition, the heat stability of a cross-linked enzyme aggregate (Dex-CLEA) formed by the dextran polyaldehyde is 3.6 times higher than that of the free enzyme.

Binay et al (Beilstein J. Org. Chem. 2016, 12, 271-277) report a highly active immobilized enzyme of FDH derived from Candida methylica. FDH is covalently immobilized on an epoxy-activated Immobead 150 carrier. The Immobead 150 carrier is firstly modified with an ethylenediamin, then activated with glutaraldehyde (FDHIGLU) and functionalized with an aldehyde group (FDHIALD). The highest immobilization and activity yields are obtained while the aldehyde-functionalized Immobead 150 is used as a carrier, which is 90% and 132%, respectively. At 35°C, the half-lives (t1/2) of free FDH, FDHI150, FDHIGLU and FDHIALD are respectively calculated to be 10.6, 28.9, 22.4 and 38.5 hours. FDHI150, FDHIGLU and FDHIALD retain 69%, 38%, and 51% of the initial activity thereof respectively after 10 times of repeated uses.

Jackon et al. (Process Biochem. Vol. 1, 9, Sep 2016, 1248-1255) report immobilization of a lactate dehydrogenase (LDH) using glyoxal-agarose. Compared to a soluble counterpart thereof, a heat stability factor obtained by the immobilized LDH is 1600 times greater.

Co-immobilization of two enzymes is also reported, such as Valikhani et al. (Biotech. Bioengg. 2018; 115:2416-2425) report co-immobilization of a cytochrome P450 monooxygenase (pigments P450s) and a glucose dehydrogenase derived from B.megaterium. Delgrove et al. (Appl.Catal.A:Gen.Vol.572, 25Feb.2019, 134-141) publish that co-immobilized Baeyer-Villiger monooxygenase (BVMO) and glucose dehydrogenase are used for synthesis of a ε-caproiactone derivative. A thermostable cyclohexanone monooxygenase (TmCHMO) from Thermocrispum municipale and a glucose dehydrogenase (GDH) (GDH-Tac) from Thermoplasma acidophilum are co-immobilized on an amino-functionalized agarose-based carrier (MANA-agarose). The co-immobilization is proved to be the most efficient biocatalyst. In the synthesis of 3,3,5-trimethylcyclohexanone, the average conversion rate is 83% in 15 repeated cycles.

In conclusion, there are still a large number of enzymes in the existing technologies that do not have effective immobilized enzyme forms, especially some enzymes that jointly participate in the same biocatalytic reaction. The co-immobilization of these enzymes to improve the recyclability of the enzymes becomes an urgent problem to be solved.

### Summary

A main purpose of the present invention is to provide a co-immobilized enzyme, a preparation method therefor and an application thereof, as to solve the problem that such enzymes are difficult to recycle in the prior art.

In order to achieve the above purpose, according to one aspect of the present invention, a co-immobilized enzyme is provided, the co-immobilized enzyme includes: an amino resin carrier, a main enzyme, and a coenzyme, there is one or more coenzymes, and the main enzyme and the coenzyme are co-immobilized on the amino resin carrier, herein the main enzyme is covalent-immobilized on the amino resin carrier, and the coenzyme is immobilized on the amino resin carrier by a mode of covalent and/or non-covalent; and the main enzyme is selected from any one of the following enzymes: transaminase, amino acid dehydrogenase, imine reductase, ketoreductase, enoyl reductase, and monooxygenase.

Further, the transaminase is derived from *B.thuringiensis* or *Vibrio fluvialis strain JS17;* preferably, the amino acid dehydrogenase is derived from *Bacillus cereus or from Bacillus sphaericus;* preferably, the imine reductase is derived from *Streptomyces sp or from Bacillus cereus;* preferably, the ketoreductase is derived from *Sporobolomyces salmonicolor* or from *Acetobacter sp.* CCTCC M209061; more preferably, the ketoreductase derived from *Acetobacter sp.* CCTCC M209061 is a mutant having the sequence of SEQ ID NO:1 or SEQ ID NO:2; preferably, the enoyl reductase is derived from *Chryseobacterium sp. CA49 or Sewanella oneidensis MR-1;* preferably, the monooxygenase is a cyclohexanone monooxygenase derived from *Rhodococcus sp. Phi1,* or from *Brachymonas petroleovorans,* or from *Rhodococcus ruber-SD1*; more preferably, the cyclohexanone monooxygenase derived from *Rhodococcus sp. Phi1* is a mutant having the sequence of SEQ ID NO:4 or SEQ ID NO:5, the cyclohexanone monooxygenase derived from *Rhodococcus ruber-SD1* is a mutant having the sequence of SEQ ID NO:7 or SEQ ID NO:8.

Further, the coenzyme is selected from at least one of the followings: lactate dehydrogenase (LDH), formate dehydrogenase (FDH), glucose dehydrogenase (GDH) and alcohol dehydrogenase (ADH); preferably, the lactate dehydrogenase is a D-lactate dehydrogenase derived from *Lactobacillus helveticus*; preferably, the formate dehydrogenase is derived from *Candida boidinii;* preferably, the glucose dehydrogenase is glucose 1-dehydrogenase derived from *Lysinibacillus sphaericus G10;* preferably, the alcohol dehydrogenase is derived from *Thermoanaerobium brockii;* and preferably, the co-immobilized enzyme is re-used for 4-25 cycles.

Further, the amino resin carrier is an amino resin carrier activated by glutaraldehyde; preferably, the amino resin carrier is an amino resin carrier with a C2 or C4 linker arm, more preferably, the amino resin carrier is selected from at least one of the followings: SEPLITE^{®}LX1000EA, LX1000HA, LX1000NH, HFA, LX1000EPN, HM100D, Purolite^{®} Lifetech^{™} ECR8309, ECR8409, ECR8305, ECR8404, ECR8315, ECR8415, HECHENG^{®}ESR-1, ESR-3, ESR-5 and ESR-8.

Further, in the co-immobilized enzyme, a mass ratio of the main enzyme and the coenzyme is 1-20:1-10; preferably, a sum of masses of the main enzyme and the coenzyme is marked as N1, a mass of the amino resin carrier is marked as N2, N1: N2 is 50-200 mg: 1 g, and preferably, 80-120 mg:1 g.

Further, either the main enzyme and the coenzyme are covalently immobilized on the amino resin carrier; or only the main enzyme is covalent-immobilized on the amino resin carrier and the coenzyme is non-covalently immobilized on the amino resin carrier by an ionic adsorption mode; preferably, the coenzyme is adsorbed on the amino resin carrier by polyethyleneimine (PEI).

Further, the coenzyme includes a first enzyme and a second enzyme, the main enzyme and the second enzyme are covalent-immobilized on the amino resin carrier, and the first enzyme is immobilized on the amino resin carrier by the ionic adsorption mode.

In a second aspect of the present application, a preparation method for any one of the above co-immobilized enzymes is provided, and the preparation method includes: activating an amino resin carrier to obtain an activated amino carrier; and covalently immobilizing a main enzyme on the activated amino carrier, and covalently and/or non-covalently immobilizing a coenzyme corresponding to the main enzyme on the activated amino carrier, to obtain the co-immobilized enzyme; herein the number of the coenzyme corresponding to the main enzyme is one or more.

Further, immobilizing the main enzyme and the coenzyme on the activated amino carrier to obtain the co-immobilized enzyme includes: mixing the main enzyme with the coenzyme, to obtain a first mixed enzyme; and immobilizing the first mixed enzyme on the activated amino carrier, to obtain the co-immobilized enzyme.

Further, the coenzyme includes a first enzyme, and immobilizing the main enzyme and the coenzyme on the activated amino carrier to obtain the co-immobilized enzyme includes: immobilizing the main enzyme on the activated amino carrier, to obtain a primary immobilized enzyme; and immobilizing the first enzyme and the primary immobilized enzyme, to obtain the co-immobilized enzyme.

Further, the coenzyme further includes a second enzyme, and immobilizing the main enzyme and the coenzyme on the activated amino carrier to obtain the co-immobilized enzyme includes: immobilizing the main enzyme and the second enzyme on the activated amino carrier, to obtain a primary immobilized enzyme; and immobilizing the first enzyme and the primary immobilized enzyme, to obtain the co-immobilized enzyme.

Further, immobilizing the first enzyme and the primary immobilized enzyme by surface coating of PEI, to obtain the co-immobilized enzyme; preferably, adding PEI to the primary immobilized enzyme until a final concentration of the PEI ranges from 0.5 w/v% to 5 w/v%, to obtain a PEI-primary immobilized enzyme; and binding the first enzyme to the PEI-primary immobilized enzyme, to obtain the co-immobilized enzyme.

Further, immobilizing the first mixed enzyme in a mass ratio of 50-150 mg:1 g on the activated amino carrier, to obtain the co-immobilized enzyme.

Further, a mass ratio of the main enzyme and the first enzyme is 1~20:1~10.

Further, a mass ratio of the main enzyme and the second enzyme is 1~20:1~10.

Further, the main enzyme is a transaminase, and the coenzyme has two co-factors, and the two factors are LDH and FDH, or LDH and GDH, and the above preparation method includes any one of the followings:
(1) mixing the transaminase with both LDH and FDH, to obtain a first enzyme mixture, and immobilizing the first enzyme mixture on an activated amino carrier, to obtain the co-immobilized enzyme;
(2) mixing the transaminase with both LDH and GDH, to obtain a second enzyme mixture, and immobilizing the second enzyme mixture on the activated amino carrier, to obtain the co-immobilized enzyme; and
(3) mixing the transaminase with LDH, to obtain a third enzyme mixture; immobilizing the third enzyme mixture on the activated amino carrier, to obtain a primary immobilized transaminase; and then performing surface coating of PEI on the primary immobilized transaminase, and immobilizing GDH or FDH, to obtain the co-immobilized enzyme.

Further, the main enzyme is an amino acid dehydrogenase, the coenzyme is FDH or GDH, and the preparation method includes any one of the followings:
(1) mixing the amino acid dehydrogenase with either FDH or GDH, to obtain an amino acid dehydrogenase mixture; and immobilizing the amino acid dehydrogenase mixture on the activated amino carrier, to obtain the co-immobilized enzyme; and
(2) immobilizing the amino acid dehydrogenase on the activated amino carrier, to obtain a primary immobilized amino acid dehydrogenase; and then performing surface coating of PEI on the primary immobilized amino acid dehydrogenase, and immobilizing GDH or FDH, to obtain the co-immobilized enzyme.

Further, the main enzyme is an imine reductase, the coenzyme is FDH or GDH, and the preparation method includes any one of the followings:
(1) mixing the imine reductase with either FDH or GDH, to obtain an imine reductase mixture; and immobilizing the imine reductase mixture on the activated amino carrier, to obtain the co-immobilized enzyme; and
(2) immobilizing the imine reductase on the activated amino carrier, to obtain a primary immobilized imine reductase; and then performing surface coating of PEI on the primary immobilized imine reductase, and immobilizing GDH or FDH, to obtain the co-immobilized enzyme.

Further, the main enzyme is a ketoreductase, the coenzyme is FDH or GDH, and the preparation method includes any one of the followings:
(1) mixing the ketoreductase with either FDH or GDH, to obtain a ketoreductase mixture; and immobilizing the ketoreductase mixture on the activated amino carrier, to obtain the co-immobilized enzyme; and
(2) immobilizing the ketoreductase on the activated amino carrier, to obtain a primary immobilized ketoreductase; and then performing surface coating of PEI on the primary immobilized ketoreductase, and immobilizing GDH or FDH, to obtain the co-immobilized enzyme.

Further, the main enzyme is an enoyl reductase, the coenzyme is FDH or GDH, and the preparation method includes any one of the followings:
(1) mixing the enoyl reductase with either FDH or GDH, to obtain a enoyl reductase mixture; and immobilizing the enoyl reductase mixture on the activated amino carrier, to obtain the co-immobilized enzyme; and
(2) immobilizing the enoyl reductase on the activated amino carrier, to obtain a primary immobilized enoyl reductase; and then performing surface coating of PEI on the primary immobilized enoyl reductase, and immobilizing GDH or FDH, to obtain the co-immobilized enzyme.

Further, the main enzyme is a cyclohexanone monoxygenase, the coenzyme is FDH or GDH, and the preparation method includes any one of the followings:
(1) mixing the cyclohexanone monoxygenase with either FDH or GDH, to obtain a monoxygenase mixture; and immobilizing the monoxygenase mixture on the activated amino carrier, to obtain the co-immobilized enzyme; and
(2) immobilizing the cyclohexanone monoxygenase on the activated amino carrier, to obtain a primary immobilized cyclohexanone monoxygenase; and then performing surface coating of PEI on the primary immobilized cyclohexanone monoxygenase, and immobilizing GDH or FDH, to obtain the co-immobilized enzyme.

Further the amino resin carrier is activated by glutaraldehyde, to obtain the activated amino carrier.

According to a third aspect of the present application, an application of any one of the above co-immobilized enzymes or the co-immobilized enzyme prepared by any one of the preparation methods for the co-immobilized enzymes in a biocatalytic reaction is provided.

Further, the biocatalytic reaction is an intermittent biocatalytic reaction or a continuous biocatalytic reaction; preferably, the co-immobilized enzyme is applied in a continuous fluidized bed or fixed bed biocatalytic reaction; and preferably, the co-immobilized enzyme is re-used for 4~25 cycles in the continuous biocatalytic reaction.

By applying a technical scheme of the present invention, the present application achieves the co-immobilization of these main enzymes and the coenzymes thereof by co-immobilizing the above main enzymes and the coenzymes thereof on the amino resin carrier, thereby it is beneficial to improve the activity and recycling efficiency of the enzyme.

### Detailed Description of the Embodiments

It should be noted that embodiments in the present application and features of the embodiments may be combined with each other in the case without conflicting. The present invention is described in detail below with reference to the embodiments.

Polyethylene imine, referred to as PEI.

In a typical embodiment of the present application, a co-immobilized enzyme is provided, and the co-immobilized enzyme includes: an amino resin carrier, a main enzyme, and a coenzyme, the main enzyme and the coenzyme are co-immobilized on the amino resin carrier, herein the main enzyme is covalent-immobilized on the amino resin carrier, and the coenzyme is immobilized on the amino resin carrier by a mode of covalent and/or non-covalent; and the main enzyme is selected from any one of the following enzymes: transaminase, amino acid dehydrogenase, imine reductase, ketoreductase, enoyl reductase, and monooxygenase.

The present application achieves the co-immobilization of these main enzymes and the coenzymes thereof by co-immobilizing the above main enzymes and the coenzymes thereof on the amino resin carrier, thereby it is beneficial to improve the activity and recycling efficiency of the enzyme.

The above various main enzymes have different specific types and activities according to different sources. In a preferred embodiment, the transaminase is derived from *B.thuringiensis* or *Vibrio fluvialis strain JS17;* preferably, the amino acid dehydrogenase is derived from *Bacillus cereus or from Bacillus sphaericus;* preferably, the imine reductase is derived from *Streptomyces sp or from Bacillus cereus;* preferably, the ketoreductase is derived from *Sporobolomyces salmonicolor* or from *Acetobacter sp.* CCTCC M209061; more preferably, the ketoreductase derived from *Acetobacter sp.* CCTCC M209061 is a mutant having the sequence of SEQ ID NO:1 or SEQ ID NO:2; preferably, the enoyl reductase is derived from *Chryseobacterium sp. CA49 or Sewanella oneidensis MR-1;* preferably, the monooxygenase is a cyclohexanone monooxygenase derived from *Rhodococcus sp. Phi1,* or from *Brachymonas petroleovorans,* or from *Rhodococcus ruber-SD1*; more preferably, the cyclohexanone monooxygenase derived from *Rhodococcus sp. Phi1* is a mutant having the sequence of SEQ ID NO:4 or SEQ ID NO:5, the cyclohexanone monooxygenase derived from *Rhodococcus ruber-SD1* is a mutant having the sequence of SEQ ID NO:7 or SEQ ID NO:8.

According to the different types of the above main enzymes, the corresponding coenzymes are selected. Preferably, these coenzymes are coenzymes capable of recycling NAD(P)+ and NADPH. In a preferred embodiment, the coenzyme is selected from at least one of the followings: lactate dehydrogenase, formate dehydrogenase, glucose dehydrogenase and alcohol dehydrogenase; preferably, the lactate dehydrogenase is a D-lactate dehydrogenase derived from *Lactobacillus helveticus*; preferably, the formate dehydrogenase is derived from *Candida boidinii;* preferably, the glucose dehydrogenase is glucose 1-dehydrogenase derived from *Lysinibacillus sphaericus G10;* preferably, the alcohol dehydrogenase is derived from *Thermoanaerobium brockii.* In another preferred embodiment, the co-immobilized enzyme is re-used for 4-25 cycles.

In the above preferred embodiment, the coenzyme corresponding to the transaminase is lactate dehydrogenase and ammonium formate dehydrogenase, or lactate dehydrogenase and glucose dehydrogenase. The coenzyme corresponding to the amino acid dehydrogenase is ammonium formate dehydrogenase or glucose dehydrogenase. The coenzyme corresponding to the imine reductase is ammonium formate dehydrogenase or glucose dehydrogenase. The coenzyme corresponding to the ketoreductase is ammonium formate dehydrogenase or glucose dehydrogenase. The coenzyme corresponding to the enoyl reductase is ammonium formate dehydrogenase or glucose dehydrogenase. The coenzyme corresponding to the monooxygenase is alcohol dehydrogenase or ammonium formate dehydrogenase or glucose dehydrogenase.

In a preferred embodiment, the lactate dehydrogenase is a D-lactate dehydrogenase (LDH for short) derived from *Lactobacillus helveticus*; preferably, the ammonium formate dehydrogenase is a formate dehydrogenase (FDH for short) derived from *Candida boidinii;* preferably, the glucose dehydrogenase is a glucose 1-dehydrogenase (GDH for short) derived from *Lysinibacillus sphaericus G10;* and preferably, the alcohol dehydrogenase is an alcohol dehydrogenase derived from *Thermoanaerobium brockii.*

There is no co-immobilized enzyme for the main enzymes and co-enzymes from each source in the above preferred embodiment in the prior art, and the co-immobilization of these main enzymes and co-enzymes is helpful to improve the recycling efficiency of these enzymes.

The carrier of the co-immobilized enzyme is an amino resin carrier, and various existing amino resin carriers may be used. In a preferred embodiment, the amino resin carrier is an amino resin carrier activated by glutaraldehyde; preferably, the amino resin carrier is an amino resin carrier with a C2 or C4 linker arm, more preferably, the amino resin carrier is selected from at least one of the followings: SEPLITE^{®}LX1000EA, LX1000HA, LX1000NH, HFA, LX1000EPN, HFA, HM100D, Purolite^{®} Lifetech^{™} ECR8309, ECR8409, ECR8305, ECR8404, ECR8315, ECR8415, HECHENG^{®}ESR-1, ESR-3, ESR-5 and ESR-8. According to the difference of the main enzyme and coenzyme to be immobilized, the different amino resin carriers may be selected.

In the above co-immobilized enzyme, the amount ratios of the main enzyme and the co-enzyme are different according to different substrates to be catalyzed. In a preferred embodiment, in the above co-immobilized enzyme, a mass ratio of the main enzyme and the coenzyme is 1-20:1-10. Preferably, a sum of masses of the main enzyme and the coenzyme is marked as N1, a mass of the amino resin carrier is marked as N2, N1: N2 is 50-200 mg: 1 g, and preferably, 80-120 mg:1 g.

The mass ratio of the main enzyme and the coenzyme is controlled within the above range, so that all the above co-immobilized enzymes of the main enzyme and the coenzyme may catalyze most of the substrates. In addition, the mass ratio of the enzyme to the carrier is controlled in the range of 50~200 mg:1 g, it may achieve the co-immobilization of all the above main enzymes and coenzymes.

In some more preferred embodiments, the mass ratio of the transaminase to the coenzyme lactate dehydrogenase thereof is 5~7:1; the mass ratio of the transaminase to the coenzyme ammonium formate dehydrogenase thereof is 5~7:1~3; the mass ratio of the transaminase to the coenzyme glucose dehydrogenase thereof is 5~7:1~2; the mass ratio of the amino acid dehydrogenase to the coenzyme ammonium formate dehydrogenase thereof is 8~10:1; the mass ratio of the amino acid dehydrogenase to the coenzyme glucose dehydrogenase thereof is 5~6:1; the mass ratio of the imine reductase to the coenzyme ammonium formate dehydrogenase thereof is 4~8;1; the mass ratio of the imine reductase to the coenzyme glucose dehydrogenase thereof is 6~8:1; the mass ratio of the ketoreductase to the coenzyme ammonium formate dehydrogenase is 4~8:1; the mass ratio of the ketoreductase to the coenzyme glucose dehydrogenase thereof is 1:3~10; the mass ratio of the enoyl reductase to the coenzyme ammonium formate dehydrogenase thereof is 6~10:1; the mass ratio of the enoyl reductase to the coenzyme glucose dehydrogenase thereof is 12~20:1; the mass ratio of the monooxygenase to the coenzyme alcohol dehydrogenase thereof is 2~3:2~3; and the mass ratio of the monooxygenase to the coenzyme glucose dehydrogenase thereof is 2~3:1. The mass ratios of the above main enzymes and the corresponding different coenzymes thereof are controlled within the above range, so that the main enzymes and the coenzymes may be configured according to the stoichiometric ratio as much as possible, thereby the catalytic activity and efficiency of the co-immobilized enzyme are improved.

In the above co-immobilized enzyme, both the main enzyme and the coenzyme are immobilized on the carrier, and a specific immobilization mode of the two on the carrier is not limited. In a preferred embodiment, both the main enzyme and the coenzyme are covalently immobilized on the amino resin carrier. In another preferred embodiment, the main enzyme is covalently immobilized on the amino resin carrier, and the coenzyme is non-covalently immobilized on the amino resin carrier by a mode of ionic adsorption; preferably, the coenzyme is adsorbed on the amino resin carrier by PEI. The above two different co-immobilization modes may be obtained by adopting different co-immobilization methods.

In the above co-immobilized enzymes, some main enzymes have only one coenzyme, and some main enzymes have two coenzymes. The co-immobilization modes of the two are the same as above, it may be the same or different, and determined according to the properties and characteristics of the specific coenzymes. In a preferred embodiment, the coenzyme includes a first enzyme and a second enzyme, the main enzyme and the second enzyme are covalently immobilized on the amino resin carrier, and the first enzyme is immobilized on the amino resin carrier by a mode of ionic adsorption. Here, the first enzyme refers to an enzyme that is sensitive to some reagents in a co-immobilization process, such as a cross-linking agent glutaraldehyde. In order to reduce the decrease of the activity of the first enzyme in the co-immobilization process, and reduce the influence on the activity of the co-immobilized enzyme and the subsequent recycling efficiency, the first enzyme is non-covalently immobilized on the amino resin carrier by the mode of ionic adsorption in the above preferred embodiment.

Transaminase TA-Bt (see Table 1 for details) and LDH and FDH are co-immobilized to a carrier LX1000HA in the optimal ratio, and the maximum number of uses may reach 15 times; the transaminase TA-Bt and LDH and GDH are co-immobilized to the carrier LX1000HA in the optimal ratio, and the maximum number of uses may reach 20~25 times; monooxygenase CHMO-Rs and GDH are co-immobilized to the carrier LX1000HA in the optimal ratio, and the maximum number of uses reaches 13 times, the monooxygenase CHMO-Rs and ADH are co-immobilized to the carrier LX1000HA in the optimal ratio, and the maximum number of uses reaches 13~15 times, they are co-immobilized to a carrier ECR8409, and the maximum number of uses reaches 16 times; mutant V1 of CHMO-Rs and ADH are co-immobilized to the carrier LX1000HA, and the maximum number of uses reaches 17 times, mutant V2 and ADH are co-immobilized to the carrier LX1000HA, and the maximum number of uses reaches 17 times; CHMO-Bp and ADH or GDH are co-immobilized to the carrier LX1000HA, and the number of uses reaches 14~15 times; CHMO-Bp mutant V1 and ADH are co-immobilized to the carrier LX1000HA, and the number of uses reaches 19 times; the CHMO-Bp mutant V1 and ADH are co-immobilized to the carrier LX1000HA, and the number of uses reaches 21 times; AADH-Bc and FDH are co-immobilized to the carrier LX1000HA in the optimal ratio, and the maximum number of uses reaches 12 times; AADH-Bs and GDH are co-immobilized to the carrier LX1000HA in the optimal ratio, and the maximum number of uses reaches 15 times; KRED-Ac and FDH are co-immobilized to the carrier ECR8409, and the maximum number of uses reaches 18 times; KRED-Ac-V1 and FDH are co-immobilized to the carrier ECR8409, and the maximum number of uses reaches 24 times; KRED-Ac and GDH are co-immobilized to the carrier ECR8409, and the maximum number of uses reaches 14 times; ERED-Sc and FDH are co-immobilized to the carrier LX1000HA in the optimal ratio, and the maximum number of uses reaches 17 times; ERED-Sc and GDH are co-immobilized to the carrier LX1000HA in the optimal ratio, and the maximum number of uses reaches 21 times; ERED-Chr is immobilized to a carrier LX1000EPN in the optimal ratio, and the maximum number of uses reaches 21 times; IRED-Str and FDH are co-immobilized to the carrier LX1000EPN in the optimal ratio, and the maximum number of uses reaches 17 times; and IRED-Bc and GDH are co-immobilized to the carrier LX1000EPN in the optimal ratio, and the maximum number of uses reaches 19 times.

In a second typical embodiment of the present application, a preparation method for any one of the above co-immobilized enzymes is provided, the preparation method includes: activating an amino resin carrier, to obtain an activated amino carrier; covalently immobilizing a main enzyme on the activated amino carrier and immobilizing a coenzyme corresponding to the main enzyme on the activated amino carrier in a mode of covalent and/or non-covalent, to obtain the co-immobilized enzyme.

In the above method for the co-immobilized enzyme, the co-immobilized enzyme may be obtained by firstly activating the amino resin carrier, and then co-immobilizing any one of the above main enzymes and the coenzymes thereof on the activated amino carrier.

For the specific method of co-immobilizing the above main enzymes and coenzymes on the activated amino carrier, appropriate steps may be selected according to the different types and properties of the coenzymes.

In a preferred embodiment, immobilizing the main enzyme and the coenzyme on the activated amino carrier to obtain the co-immobilized enzyme includes: mixing the main enzyme with the coenzyme, to obtain a first mixed enzyme; and immobilizing the first mixed enzyme on the activated amino carrier, to obtain the co-immobilized enzyme.

In this preferred method, regardless of the number of the coenzymes, the coenzyme may be immobilized on the carrier after being mixed with the main enzyme. According to the different main enzymes and coenzymes, the mixing ratios are different. Preferably, according to the previous mass ratio 1~20:1~10 of the main enzyme and the coenzyme, the main enzyme is mixed with the coenzyme, to obtain the first mixed enzyme; and according to the previous ratio of N1/N2, the first mixed enzyme is immobilized on the activated amino carrier, to obtain the co-immobilized enzyme.

In a preferred embodiment, the coenzyme includes a first enzyme, and immobilizing the main enzyme and the coenzyme on the activated amino carrier to obtain the co-immobilized enzyme includes: immobilizing the main enzyme on the activated amino carrier, to obtain a primary immobilized enzyme; and immobilizing the first enzyme and the primary immobilized enzyme, to obtain the co-immobilized enzyme.

While there is only one coenzyme, the above method of immobilizing together with the main enzyme after mixing may be adopted, or the above method of step-by-step immobilizing may be adopted. For the co-immobilization of some coenzymes sensitive to the glutaraldehyde in the step of activating the amino carrier, a step-by-step immobilization method is preferentially adopted, this may retain the activity of the coenzyme to a greater extent, thereby the recycling efficiency of the co-immobilized enzyme is improved.

In a preferred embodiment, the coenzyme further includes a second enzyme, and immobilizing the main enzyme and the coenzyme on the activated amino carrier to obtain the co-immobilized enzyme includes: co-immobilizing the main enzyme and the second enzyme on the activated amino carrier, to obtain a primary immobilized enzyme; and immobilizing the first enzyme and the primary immobilized enzyme, to obtain the co-immobilized enzyme.

While there is more than one coenzyme, according to the sensitivities of the coenzyme and glutaraldehyde and other activators, the above part may be used to mix with the main enzyme and then immobilized on the activated amino carrier. In the second step, the highly sensitive coenzyme is immobilized on the carrier, thus the activity of the highly sensitive enzyme may be improved, thereby the overall activity and recycling efficiency of the prepared co-immobilized enzyme are improved. In the above preferred embodiment, the first enzyme is an enzyme that is relatively sensitive to the glutaraldehyde, and the sensitivity of the second enzyme is relatively low, so the second enzyme and the main enzyme are mixed and immobilized, and then the first enzyme is separately immobilized.

In the above step-by-step immobilization step, the second time of immobilization is performed for the purpose of improving the activity of the coenzyme to be immobilized. Therefore, any modes that may improve the activity of this type of the coenzymes and may achieve the immobilization to it are applicable to the present application. In a preferred embodiment, the first enzyme is immobilized with the primary immobilized enzyme through PEI, to obtain the co-immobilized enzyme. In other preferred embodiments, PEI is added to the primary immobilized enzyme until a final concentration (mass volume concentration) of PEI is 0.5%~5%, to obtain a PEI-primary immobilized enzyme; and the first enzyme is added to the PEI-primary immobilized enzyme for incubation, to obtain the co-immobilized enzyme.

PEI is polyethylene imine, it may be used as a support in the non-solid form, and it adsorbs the coenzyme to be immobilized on the carrier by the mode of ionic adsorption. The specific amount of PEI added may be reasonably adjusted according to actual needs. While the final concentration of PEI added is 0.5%~5%, the immobilization of all types of the coenzymes described above may be achieved.

While the main enzyme is the transaminase, the coenzyme has two cofactors, LDH and FDH, or LDH and GDH, and the preparation method thereof includes a one-step immobilization method or a step-by-step immobilization method. The one-step immobilization method is to obtain a first enzyme mixture or a second enzyme mixture by mixing the two cofactors in different combinations with the transaminase respectively, and then immobilize the first enzyme mixture or the second enzyme mixture on the activated amino carrier, to obtain the co-immobilized enzyme. Step-by-step immobilization method: mixing the transaminase with LDH, to obtain a third enzyme mixture; immobilizing the third enzyme mixture on the activated amino carrier, to obtain a primary immobilized transaminase; and through a mode of performing surface coating of PEI on the primary immobilized transaminase, immobilizing GDH or FDH, to obtain the co-immobilized enzyme.

While the main enzyme is the amino acid dehydrogenase, and the coenzyme is FDH or GDH, the preparation method includes any one of the followings: mixing the amino acid dehydrogenase with FDH or GDH, to obtain an amino acid dehydrogenase mixture; immobilizing the amino acid dehydrogenase mixture on the activated amino carrier, to obtain the co-immobilized enzyme; or, immobilizing the amino acid dehydrogenase on the activated amino carrier, to obtain a primary immobilized amino acid dehydrogenase; through a mode of performing surface coating of PEI on the primary immobilized amino acid dehydrogenase, immobilizing GDH or FDH, to obtain the co-immobilized enzyme.

While the main enzyme is the imine reductase, and the coenzyme is FDH or GDH, the preparation method includes any one of the followings: mixing the imine reductase, and FDH or GDH, to obtain an imine enzyme mixture; immobilizing the imine enzyme mixture on the activated amino carrier, to obtain the co-immobilized enzyme; or, immobilizing the imine reductase on the activated amino carrier, to obtain a primary immobilized imine reductase; and through a mode of performing surface coating of PEI on the primary immobilized imine reductase, immobilizing GDH or FDH, to obtain the co-immobilized enzyme.

While the main enzyme is the ketoreductase, and the coenzyme is FDH or GDH, the preparation method includes any one of the followings: mixing the ketoreductase, and FDH or GDH, to obtain a ketoreductase mixture; immobilizing the ketoreductase mixture on the activated amino carrier, to obtain the co-immobilized enzyme; or immobilizing the ketoreductase on the activated amino carrier, to obtain a primary immobilized ketoreductase; and through a mode of performing surface coating of PEI on the primary immobilized ketoreductase, immobilizing GDH or FDH, to obtain the co-immobilized enzyme.

While the main enzyme is the enoyl reductase, and the coenzyme is FDH or GDH, the preparation method includes any one of the followings: mixing the enoyl reductase, and FDH or GDH, to obtain an enoyl reductase mixture; immobilizing the enoyl reductase mixture on the activated amino carrier, to obtain the co-immobilized enzyme; or, immobilizing the enoyl reductase on the activated amino carrier, to obtain a primary immobilized enoyl reductase; and through a mode of performing surface coating of PEI on the primary immobilized enoyl reductase, immobilizing GDH or FDH, to obtain the co-immobilized enzyme.

While the main enzyme is the cyclohexanone monooxygenase, and the coenzyme is FDH or GDH, the preparation method includes any one of the followings: mixing the cyclohexanone monooxygenase, and FDH or GDH, to obtain a monooxygenase mixture; immobilizing the monooxygenase mixture on the activated amino carrier, to obtain the co-immobilized enzyme; or, immobilizing the cyclohexanone monooxygenase on the activated amino carrier, to obtain a primary immobilized cyclohexanone monooxygenase; and through a mode of performing surface coating of PEI on the primary immobilized cyclohexanone monooxygenase, immobilizing GDH or FDH, to obtain the co-immobilized enzyme.

In the above preparation methods for the co-immobilization of various main enzymes and coenzymes thereof, the PEI surface coating method is the same as the previous operation of "adding PEI to the primary immobilized enzyme until the final concentration of PEI is 0.5w/v%~ 5w/v %, to obtain the PEI-primary immobilized enzyme complex; and combining the first enzyme with the PEI-primary immobilized enzyme complex, to obtain the co-immobilized enzyme", it is not repeatedly described here.

In the above step of activating the amino carrier, an existing activator may be used for activation. In a preferred embodiment, the glutaraldehyde is used to activate the amino resin carrier, to obtain the activated amino carrier. The glutaraldehyde has a wide range of applications and is the most common.

In the above co-immobilization step, according to the different co-immobilization methods and the different types and numbers of the co-immobilized coenzymes, the ratios of the total mass of the co-immobilized coenzymes to the total mass of the activated amino carriers are also different. Correspondingly, the mass ratios of the main enzymes to the coenzymes are also different, and while there are two types of the coenzymes, the mass ratios of the main enzymes to the first coenzyme and the second coenzyme are also different, and it may be reasonably adjusted according to actual needs.

In a preferred embodiment, the first mixed enzyme is immobilized on the activated amino carrier at the mass ratio of 50~150 mg:1 g, to obtain the co-immobilized enzyme.

In a preferred embodiment, the mass ratio of the main enzyme to the first enzyme is 1~20:1~ 10.

In a preferred embodiment, the mass ratio of the main enzyme to the second enzyme is 1~ 20:1~10.

Preferably, in the above co-immobilization step, the mass ratio of the transaminase to the coenzyme lactate dehydrogenase thereof is 5~7:1; the mass ratio of the transaminase to the coenzyme ammonium formate dehydrogenase thereof is 5~7:1~3; the mass ratio of the transaminase to the coenzyme glucose dehydrogenase thereof is 5~7:1~2; the mass ratio of the amino acid dehydrogenase to the coenzyme ammonium formate dehydrogenase thereof is 8~10:1; the mass ratio of the amino acid dehydrogenase to the coenzyme glucose dehydrogenase thereof is 4 ~ 6:1; the mass ratio of the imine reductase to the coenzyme ammonium formate dehydrogenase thereof is 4~6;1; the mass ratio of the imine reductase to the coenzyme glucose dehydrogenase thereof is 5~6:1; the mass ratio of the ketoreductase to the coenzyme ammonium formate dehydrogenase is 4~6:1; the mass ratio of the ketoreductase to the coenzyme glucose dehydrogenase thereof is 1:5~10; the mass ratio of the enoyl reductase to the coenzyme ammonium formate dehydrogenase thereof is 6~8:1; the mass ratio of the enoyl reductase to the coenzyme glucose dehydrogenase thereof is 18~20:1; the mass ratio of the monooxygenase to the coenzyme alcohol dehydrogenase thereof is 2~3:2~3; and the mass ratio of the monooxygenase to the coenzyme glucose dehydrogenase thereof is 2~3:1.

The mass ratios of the above main enzymes and the corresponding different coenzymes thereof are controlled within the above range, so that the main enzymes and the coenzymes may be configured according to the stoichiometric ratio as much as possible, thereby the catalytic activity and efficiency of the co-immobilized enzyme are improved.

In a third typical embodiment of the present application, an application of any one of the above co-immobilized enzymes or the co-immobilized enzyme prepared by any one of the preparation methods for the co-immobilized enzymes in a biocatalytic reaction is provided. Preferably, the biocatalytic reaction is a continuous biocatalytic reaction.

The beneficial effects of the present application are further described below with reference to the specific embodiments.

The enzymes used in the following embodiments and sources thereof are shown in Table 1 below, and Tables 2 to 4 show sequences of some enzymes.

**Table 1:**

| Enzyme | Short name | Species source |
|---|---|---|
| D-amino acid transaminase | TA-Bt | *B.thuringiensis* |
| Pyruvate transaminase | TA-Vf | *Vibrio fluvialis strain JSI7* |
| Ketoreductase | KRED-Ss | *Sporobolomyces salmonicolor* |
| | KRED-Ac | *Acetobacter sp. CCTCC M209061* |
| Alcohol dehydrogenase | ADH-Tb | *Thermoanaerobium brockii* |
| D-lactate dehydrogenase | LDH | *Lactobacillus helveticus* |
| Ammonium formate dehydrogenase | FDH | *Candida boidinii* |
| Glucose 1-dehydrogenase | GDH | *Lysinibacillus sphaericus G10* |
| Cyclohexanone monooxygenase | CHMO-Rs | *Rhodococcus sp. Phil* |
| | CHMO-Bp | *Brachymonas petroleovorans* |
| | CHMO-Rr | *Rhodococcus ruber-SDI* |
| Enoyl reductase | ERED-Sc | *Saccharomyces cerevisiae* |
| | ERED-Chr | *Chryseobacterium sp. CA49* |
| Imine reductase | IRED-Str | *Streptomyces sp.* |
| | IRED-Bc | *Bacillus cereus* |
| Leucine dehydrogenase | AADH-Bc | *Bacillus cereus* |
| Phenylalanine dehydrogenase | AADH-Bs | *Bacillus sphaericus* |

**Table 2:**

| KRED-Ac | Sequence number | Sequence |
|---|---|---|
| Female parent | SEQ ID NO:3 | MARVAGKVAIVSGAANGIGKATAQLLAKEGAKVVI GDLKEEDGQKAVAEIKAAGGEAAFVKLNVTDEAAWKA AIGQTLKLYGRLDIAVNNAGINYSGSVESTSLEDWRRV QSINLDGVFLGTQVAIEAMKKSGGGSIVNLSSISGLIGD PMLAAYVASKGGVRLFTKSAALHCAKSGYKIRVNSVHP GYIWTPMVAGLTKEDAAARQKLVDLHPIGHLGEPNDIA YGILYLASDESKFVTGSELVIDGGYTAQ |
| Mutant 1(KRED-Ac-V1) | SEQ ID NO:2 | E144S+A94N+N156V |
| Mutant 2(KRED-AC-V2) | SEQ ID NO:1 | E144S+A94T+N156T |

**Table 3:**

| CHMO-Rs | Sequence number | Sequence |
|---|---|---|
| Female parent | SEQ ID NO:6 | MTAQISPTVVDAVVIGAGFGGIYAVHKLHNEQGL TVVGFDKADGPGGTWYWNRYPGALSDTESHLYRFS FDRDLLQDGTWKTTYITQPEILEYLESVVDRFDLRRH |
| | | FRFGTEVTSAIYLEDENLWEVSTDKGEVYRAKYVVN AVGLLSAINFPDLPGLDTFEGETIHTAAWPEGKNLAG KRVGVIGTGSTGQQVITALAPEVEHLTVFVRTPQYSV PVGNRPVTKEQIDAIKADYDGIWDSVKKSAVAFGFE ESTLPAMSVSEEERNRIFQEAWDHGGGFRFMFGTF GDIATDEAANEAAASFIRSKIAEIIEDPETARKLMPTGL YAKRPLCDNGYYEVYNRPNVEAVAIKENPIREVTAK GVVTEDGVLHELDVLVFATGFDAVDGNYRRIEIRGR NGLHINDHWDGQPTSYLGVTTANFPNWFMVLGPNG PFTNLPPSIETQVEWISDTVAYAERNEIRAIEPTPEAE EEWTQTCTDIANATLFTRGDSWIFGANVPGKKPSVL FYLGGLGNYRNVLAGVVADSYRGFELKSAVPVTA |
| Mutant 1 (CHMO-R s-Cv-V1) | SEQ ID NO:5 | F508Y+F435N+L438A+T436S+F280V+S441V |
| Mutant 2 (CHMO-R s-V2) | SEQ ID NO:4 | F508Y+F435N+L438A+T436S+F280V+S441V+L510V |

**Table 4:**

| CHMO-Rr | Sequence number | Sequence |
|---|---|---|
| Female parent | SEQ ID NO:9 | MTTSIDREALRRKYAEERDKRIRPDGNDQYIRLD HVDGWSHDPYMPITPREPKLDHVTFAFIGGGFSGLV TAARLRESGVESVRIIDKAGDFGGVWYWNRYPGAM CDTAAMVYMPLLEETGYMPTEKYAHGPEILEHCQRI GKHYDLYDDALFHTEVTDLVWQEHDQRWRISTNRG DHFTAQFVGMGTGPLHVAQLPGIPGIESFRGKSFHT SRWDYDYTGGDALGAPMDKLADKRVAVIGTGATAV QCVPELAKYCRELYVVQRTPSAVDERGNHPIDEKW FAQIATPGWQKRWLDSFTAIWDGVLTDPSELAIEHE DLVQDGWTALGQRMRAAVGSVPIEQYSPENVQRAL EEADDEQMERIRARVDEIVTDPATAAQLKAWFRQM |
| | | CKRPCFHDDYLPAFNRPNTHLVDTGGKGVERITENG VVVAGVEYEVDCIVYASGFEFLGTGYTDRAGFDPTG RDGVKLSEHWAQGTRTLHGMHTYGFPNLFVLQLMQ GAALGSNIPHNFVEAARVVAAIVDHVLSTGTSSVETT KEAEQAWVQLLLDHGRPLGNPECTPGYYNNEGKPA ELKDRLNVGYPAGSAAFFRMMDHWLAAGSFDGLTF R |
| Mutant 1(CHMO-Rr-V1) | SEQ ID NO:8 | P190L+Y559M+C249V+C393V+C257A+M45T |
| Mutant 2(CHM0-Rr-V2) | SEQ ID NO:7 | Y559M+P190L+P504V |

PB in the following embodiments represents a phosphate buffer.

### Embodiment 1

4~5 mL of 0.1 M phosphate buffer (pH 7.5) is used to wash 1 g of an amino resin, 4 mL of 0.1M phosphate buffer (pH 7.5) is used to resuspend, 25%~50% (w/v) of glutaraldehyde aqueous solution is dropwise added, so that a final concentration of glutaraldehyde is 2% (w/v), and it is incubated for 1 hour at 20°C with gentle shaking, filtered and washed for 3 times with 0.1 M phosphate buffer (pH 7.5).

4 mL of enzyme solution (the appropriate ratio of TA and LDH is adjusted) containing 100~120 mg of a protein is added to the glutaraldehyde-activated resin, and after being incubated at 20~ 25°C with gentle shaking, it is filtered and washed with 0.1 M phosphate buffer (pH 7.5) for 3 times.

The more stable linkage may be achieved by reducing an imine double-bond with a borohydride. 1 g of an immobilized enzyme is resuspended with 4 mL of a buffer (50 mM NaHCO₃-Na₂CO₃, pH 8.0~10.0), and NaBH₄ is added at 5~15°C, so that a final concentration of NaBH4 is 1 mg/mL. After being stirred for 1-2 hours at 5-15°C, it is filtered and washed with 0.1 M phosphate buffer (pH 7.5) for 3 times.

The co-immobilization activity of TA and LDH is tested with free FDH to achieve regeneration of NADH. It is testedwith the following substrate 1:

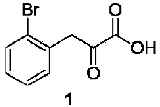

5 mL of 0.1 M phosphate buffer (pH 8.0) is added to a 10 mL reactor, and then 100 mg of the above substrate 1, 80 mg of an ammonium formate (FDH), and 5 mg of PLP are added, pH is adjusted to 7.5-8.0, then 5 mg of NAD+ and 30 mg of FDH free enzyme are added, to obtain 100 mg of the co-immobilized enzyme (wet enzyme, containing 50-80% of water). It is reacted at 30°C for 16-20 hours, and the conversion rate is detected by a High Performance Liquid Chromatography (HPLC), and results are shown in the following table.

**Table 5:**

| Co-immobilized enzyme | Carrier | TA:LDH mass ratio | Evaluation | Conversion rate (%) | Number of cycles |
|---|---|---|---|---|---|
| TA-Bt+LDH | LX1000HA | 7:1 | No reduction step | >99% | 15 |
| TA-Bt+LDH | LX1000HA | 7:1 | Reductio n step | >99% | 17 |
| TA-Vf+LDH | LX1000HA | 5:1 | No reduction step | >99% | 12 |
| TA-Vf+LDH | LX1000HA | 5:1 | Reductio n step | >99% | 13 |
| TA-Bt+LDH | ECR8309 | 7:1 | No reduction step | >99% | 7 |
| TA-Bt+LDH | HFA | 7:1 | No reduction step | >99% | 10 |
| TA-Bt+LDH | ECR8315 | 7:1 | No reduction step | >99% | 7 |
| TA-Bt+LDH | ESR-1 | 7:1 | No reduction step | >99% | 8 |
| TA-Vf+LDH | ECR8309 | 5:1 | No reduction step | >99% | 6 |
| TA-Vf+LDH | HFA | 5:1 | No reduction step | >99% | 9 |
| TA-Vf+LDH | ECR8315 | 5:1 | No reduction step | >99% | 8 |
| TA-Vf+LDH | ESR-1 | 5:1 | No reduction step | >99% | 8 |

### Embodiment 2: Co-immobilization of TA-Bt, LDH and FDH

### Method 1: One-step co-immobilization

1-2 mL of 0.1 M PB (PH 7.5) is used to wash 1 g of an amino resin, 4 mL of 0.1 M PB (PH 7.5) is used to resuspend, and glutaraldehyde aqueous solution with a concentration of 25(w/v)%~50(w/v)% is dropwise added to resuspension so that a final concentration of glutaraldehyde is 2(w/v)%. It is incubated for 1 hour at 20°C with gentle shaking, then filtered and washed with 0.1 M PB (pH 7.5) for 3 times. 4 mL of enzyme solution (the appropriate ratio of TA and LDH and FDH is adjusted) containing 100 ~ 120 mg of a protein is added to the glutaraldehyde-activated resin, and after being incubated at 20~25°C with gentle shaking, it is filtered and washed with 0.1 M phosphate buffer (pH 7.5) for 3 times.

### Method 2: Two-step co-immobilization

In order to improve the activity and stability of FDH, 1 g of co-immobilized TA and LDH is resuspended with 0.1 M PB (pH 7.0-7.5), and PEI solution (final concentration 2%) is added, and then 20 mg of FDH is added. After being incubated at 20-25°C with gentle shaking, it is filtered and washed with 0.1 M PB (pH 7.5) for 3 times.

The activities of co-immobilized TA, LDH and FDH are detected by the following reactions:
5 mL of 0.1 M phosphate buffer (pH 8.0) is added to a 10 mL reactor, and then 100 mg of the above substrate 1, 80 mg of FDH, and 5 mg of PLP are added, pH is adjusted to 7.5-8.0, then 5 mg of NAD+ and 100 mg of the co-immobilized enzyme (wet, containing 50-80% of water) are added. It is reacted at 30°C for 16-20 hours, and the conversion rate is tested. Test results are shown in the following table.

**Table 6:**

| Co-immobilized enzyme | Carrier | Method | TA:LDH:FDH mass ratio | Conversion rate (%) | Number of cycles |
|---|---|---|---|---|---|
| TA-Bt+LDH+FDH | LX1000HA | Method 1 | 7:1:2 | >99% | 6 |
| TA-Bt+LDH+FDH | LX1000HA | Method 1 | 5:1:1 | >99% | 5 |
| TA-Bt+LDH+FDH | LX1000HA | Method 1 | 7:1:1 | >99% | 6 |
| TA-Bt+LDH+FDH | LX1000HA | Method 2 | 7:1:2 | >99% | 15 |
| TA-Bt+LDH+FDH | LX1000HA | Method 2 | 5:1:1 | >99% | 12 |
| TA-Bt+LDH+FDH | LX1000HA | Method 2 | 6:1:2 | >99% | 14 |
| TA-Vf+LDH+FDH | LX1000HA | Method 1 | 5:1:3 | >99% | 5 |
| TA-Vf+LDH+FDH | LX1000HA | Method 1 | 5:1:1 | >99% | 4 |
| TA-Vf+LDH+FDH | LX1000HA | Method 2 | 5:1:3 | >99% | 7 |

### Embodiment 3: Co-immobilization of TA-Bt, LDH and GDH

### Method 1 and Method 2 are the same as Embodiment 2 except that GDH is used instead of FDH.

The activities of co-immobilized TA, LDH and GDH are detected by the following reactions:
5 mL of 0.1 M phosphate buffer (pH 8.0) is added to a 10 mL reactor, and then 100 mg of the above substrate 1, 120 mg of glucose and 5 mg of PLP are added, pH is adjusted to 7.5-8.0, then 5 mg of NAD+ and 100 mg of the co-immobilized enzyme (wet, containing 50-80% of water) are added. It is reacted at 30°C for 16-20 hours, and the conversion rate is tested. Test results are shown in Table 7.

**Table 7:**

| Co-immobilized enzyme | Carrier | Evaluation | TA:LDH:GDH mass ratio | Conversion rate (%) | Number of cycles |
|---|---|---|---|---|---|
| TA-Bt+LDH+GDH | LX1000HA | Method 1 | 7:1:2 | >99% | 12 |
| TA-Bt+LDH+GDH | LX1000EA | Method 1 | 7:1:1 | >99% | 5 |
| TA-Bt+LDH+GDH | LX1000NH | Method 1 | 7:1:2 | >99% | 6 |
| TA-Bt+LDH+GDH | LX1000EPN | Method 1 | 7:1:2 | >99% | 9 |
| TA-Bt+LDH+GDH | HFA | Method 1 | 7:1:2 | >99% | 10 |
| TA-Bt+LDH+GDH | HM100D | Method 1 | 7:1:2 | >99% | 6 |
| TA-Bt+LDH+GDH | ECR8309 | Method 1 | 7:1:2 | >99% | 5 |
| TA-Bt+LDH+GDH | ECR8409 | Method 1 | 7:1:2 | >99% | 13 |
| TA-Bt+LDH+GDH | ECR8305 | Method 1 | 7:1:2 | >99% | 6 |
| TA-Bt+LDH+GDH | ECR8404 | Method 1 | 7:1:2 | >99% | 6 |
| TA-Bt+LDH+GDH | ECR8315 | Method 1 | 7:1:2 | >99% | 7 |
| TA-Bt+LDH+GDH | ECR8415 | Method 1 | 7:1:2 | >99% | 8 |
| TA-Bt+LDH+GDH | ESR-1 | Method 1 | 7:1:2 | >99% | 7 |
| TA-Bt+LDH+GDH | ESR-3 | Method 1 | 7:1:2 | >99% | 8 |
| TA-Bt+LDH+GDH | ESR-5 | Method 1 | 7:1:2 | >99% | 6 |
| TA-Bt+LDH+GDH | ESR-8 | Method 1 | 7:1:2 | >99% | 7 |
| TA-Bt+LDH+GDH | LX1000HA | Method 2 | 7:1:2 | >99% | 20-25 |
| TA-Bt+LDH+GDH | ECR8409 | Method 2 | 7:1:1 | >99% | 20-25 |
| TA-Vf+LDH+GDH | LX1000HA | Method 1 | 5:1:2 | >99% | 8 |
| TA-Vf+LDH+GDH | LX1000HA | Method 1 | 5:1:1 | >99% | 7 |
| TA-Vf+LDH+GDH | LX1000HA | Method 2 | 5:1:2 | >99% | 14 |

### Embodiment 4: Co-immobilization of CHMO and ADH and GDH

Method 1 and Method 2 in Embodiment 2 are used, and the remaining steps are the same except that the main enzyme and coenzyme are replaced by CHMO, ADH, and GDH.

The activity of CHMO, ADH and GDH co-immobilized enzyme is detected by reacting with the following substrate 2:

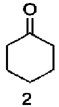

The activity of CHMO and GDH co-immobilized enzyme is detected by the following reaction:
3 mL of 0.1 M PB (pH 8.0) is loaded in a 10 ml reaction flask, and then, 50 mg of the substrate 2, 100 mg of glucose and 5 mg of NADP+ are added, and 200-300 mg of CHMO and GDH co-immobilized enzyme (wet, containing 50~80% of water) is added. It is reacted at 30°C for 16-20 hours, and the conversion rate is tested.

The activity of CHMO and ADH co-immobilized enzyme is detected by the following reaction:
0.3 mL of isopropanol is loaded in a 10 mL reaction flask, and then 500 mg of the substrate 2 is added, and 3 mL of 0.1 M PB (pH 8.0) containing 5 mg of NADP+ and 100-200 mg of CHMO and ADH co-immobilized enzyme (wet, containing 50-80% of water) are added. It is reacted at 30°C for 16-20 hours, and the conversion rate is tested. Test results are shown in Table 8.

**Table 8:**

| Co-immobilized enzyme | Carrier | CHMO:GDH/ADH mass ratio | Conversion rate (%) | Number of cycles |
|---|---|---|---|---|
| CHMO-Rs+GDH | LX1000HA | 3:1 | >90% | 13 |
| | LX1000HA | 2:1 | >90% | 7 |
| CHMO-Rs+ADH | LX1000HA | 1:1 | >90% | 6 |
| | LX1000HA | 2:3 | >90% | 5 |
| | LX1000HA | 3:2 | >90% | 12 |
| CHMO-Rs-VI+ADH | LX1000HA | 3:1 | >90% | 17 |
| CHM0-RS-V2+ADH | LX1000HA | 3:1 | >90% | 20 |
| CHMO-Bp+GDH | LX1000HA | 2 : 1 | >90% | 7 |
| | LX1000HA | 3:1 | >90% | 15 |
| | LX1000HA | 1.5:1 | >90% | 6 |
| CHMO-Bp+ADH | LX1000HA | 2:1 | >90% | 7 |
| | LX1000HA | 3:1 | >90% | 14 |
| | LX1000HA | 1.5:1 | >90% | 6 |
| CHMO-Bp-VI+ADH | LX1000HA | 3:1 | >90% | 19 |
| CHMO-Bp-V2+ADH | LX1000HA | 3:1 | >90% | 21 |

### Embodiment 5: Co-immobilization of AADH and FDH/GDH

### Method 1:

The remaining steps are the same as Method 1 in Embodiment 2, except that the enzymes are different.

### Method 2:

1-2 mL of 0.1 M PB (PH 7.5) is used to wash 1 g of an amino resin, and 4 mL of 0.1 M PB (PH 7.5) is used to resuspend; and glutaraldehyde aqueous solution with a concentration of 25%~50% is dropwise added to resuspension so that a final concentration of glutaraldehyde is 2%. It is incubated for 1 hour at 20°C with gentle shaking, then filtered and washed with 0.1 M PB (pH 7.5) for 3 times.

4 mL of enzyme solution containing 50~100 mg of a protein (only AADH) is added to the glutaraldehyde-activated resin, and after being incubated at 20~25°C with gentle shaking, it is filtered and washed with 0.1 M phosphate buffer (pH 7.5) for 3 times. After being resuspended with 0.1 M PB (pH 7.0-7.5), PEI solution (final concentration 2%) is added, and 20-50 mg of GDH/FDH is added, after being incubated at 20-25°C with gentle shaking, it is filtered and washed with 0.1 M PB (pH 7.5) for 3 times.

The activity of AADH and FDH co-immobilized enzyme is detected by reactions of the following substrates:

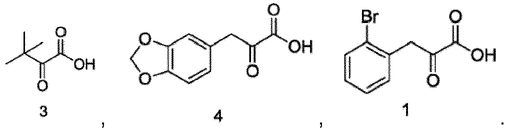

5 mL of 0.1 M Tris-CI buffer (pH 8.0-9.0) is added to a 10 mL reactor, and then 100 mg of the substrate 3 or 4 or 1, and 108 mg of an ammonium chloride are added, pH is adjusted to 7.5-8.0, and then 10-50 mg of NAD⁺, 80 mg of an ammonium formate and 100 mg of the co-immobilized enzyme are added. After it is reacted at 30°C for 16-20 hours, a conversion experiment is performed.

An activity detection method of AADH and FDH co-immobilized enzyme is as follows:
5 mL of 0.1 M Tris-CI buffer (pH 8.0-9.0) is added to a 10 mL reactor, and then 100 mg of the substrate 5 or 6 or 7, and 108 mg of an ammonium chloride are added, pH is adjusted to 7.5-8.0, and then 10-50 mg of NAD+, 150 mg of glucose and 100 mg of the co-immobilized enzyme are added. After it is reacted at 30°C for 16-20 hours, a conversion experiment is performed. Detection results are shown in Table 9.

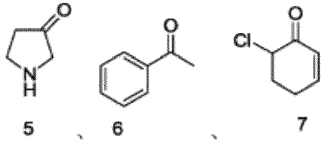

**Table 9:**

| Co-immobilized enzyme | Carrier | Method | AADH : GDH/ADH | Conversion rate (%) | Number of cycles |
|---|---|---|---|---|---|
| AADH-Bc+FDH | LX1000EA | Method 1 | 8:1 | >99% | 4 |
| AADH-Bc+FDH | LX1000EPA | Method 1 | 8:1 | >99% | 5 |
| AADH-Bc+FDH | LX1000HFA | Method 1 | 10:1 | >99% | 7 |
| AADH-Bc+FDH | ECR8415 | Method 1 | 8:1 | >99% | 4 |
| AADH-Bc+FDH | ESR-3 | Method 1 | 8:1 | >99% | 5 |
| AADH-Bc+FDH | LX1000HA | Method 1 | 8:1 | >99% | 5 |
| AADH-Bc+FDH | LX1000HA | Method 1 | 10:1 | >99% | 6 |
| AADH-Bc+FDH | LX1000HA | Method 2 | 8:1 | >99% | 11 |
| AADH-Bc+FDH | LX1000HA | Method 2 | 10:1 | 99% | 12 |
| AADH-Bs+GDH | ECR8309 | Method 1 | 5:1 | >99% | 4 |
| AADH-Bs+GDH | ECR8409 | Method 1 | 5:1 | >99% | 7 |
| AADH-Bs+GDH | ECR8315 | Method 1 | 5:1 | >99% | 6 |
| AADH-Bs+GDH | ESR-1 | Method 1 | 5:1 | >99% | 5 |
| AADH-Bs+GDH | LX1000HA | Method 1 | 5:1 | >99% | 7 |
| AADH-Bs+GDH | LX1000HA | Method 1 | 6:1 | >99% | 7 |
| AADH-Bs+GDH | LX1000HA | Method 2 | 5:1 | >99% | 9 |
| AADH-Bs+GDH | LX1000HA | Method 2 | 6:1 | >99% | 15 |
| AADH-Bs+GDH | LX1000HA | Method 2 | 4:1 | >99% | 8 |

### Embodiment 6: Co-immobilization of KRED and FDH/GDH

Except that the enzymes are different, the remaining steps of Methods 1 and 2 are the same as Embodiment 5.

The activity of KRED and FDH co-immobilized enzyme is detected by reactions of the following substrate 5 or 6:
3 mL of 0.1 M PB (pH 7.0-8.0) is loaded in a 10 mL reactor, and then 100 mg of the substrate 5 or 6 is added, and 10-50 mg of NAD(P)+, 80 mg of an ammonium formate and 100 mg of the co-immobilization enzyme are added. It is reacted at 30°C for 16-20 hours, and the conversion rate is tested.

The activity of KRED and GDH co-immobilized enzyme is detected by the following reaction:
3 mL of 0.1 M PB (pH 7.0-8.0) is loaded in a 10 mL reactor, and then 100 mg of the substrate 5 or 6 is added, and 10-50 mg of NAD(P)+, 120 mg of glucose and 100 mg of the co-immobilized enzyme are added. It is reacted at 30°C for 16-20 hours, and the conversion rate is tested.

Test results are shown in Table 10.

| Co-immobilized enzyme | Carrier | Method | KRED:GDH/FDH mass ratio | Conversion rate (%) | Number of cycles |
|---|---|---|---|---|---|
| KRED-Ac+FDH | LX1000HA | Method 1 | 4:1 | >99% | 4 |
| KRED-Ac+FDH | LX1000HA | Method 1 | 6:1 | >99% | 6 |
| KRED-Ac+FDH | HFA | Method 1 | 6:1 | >99% | 4 |
| KRED-Ac+FDH | HFA | Method 1 | 8:1 | >99% | 5 |
| KRED-Ac+FDH | LX1000EA | Method 1 | 6:1 | >99% | 4 |
| KRED-Ac+FDH | LX1000NH | Method 1 | 6:1 | >99% | 4 |
| KRED-Ac+FDH | LX1000EPN | Method 1 | 6:1 | >99% | 5 |
| KRED-Ac+FDH | HM100D | Method 1 | 6:1 | >99% | 4 |
| KRED-Ac+FDH | ECR8309 | Method 1 | 6:1 | >99% | 4 |
| KRED-Ac+FDH | ECR8409 | Method 1 | 6:1 | >99% | 11 |
| KRED-Ac+FDH | ECR8305 | Method 1 | 6:1 | >99% | 4 |
| KRED-Ac+FDH | ECR8404 | Method 1 | 6:1 | >99% | 5 |
| KRED-Ac+FDH | ECR8315 | Method 1 | 6:1 | >99% | 5 |
| KRED-Ac+FDH | ECR8415 | Method 1 | 6:1 | >99% | 5 |
| KRED-Ac+FDH | ESR-1 | Method 1 | 6:1 | >99% | 4 |
| KRED-Ac+FDH | ESR-3 | Method 1 | 6:1 | >99% | 5 |
| KRED-Ac+FDH | ESR-5 | Method 1 | 6:1 | >99% | 5 |
| KRED-Ac+FDH | LX1000HA | Method 2 | 4:1 | >99% | 12 |
| KRED-Ac+FDH | HFA | Method 2 | 6:1 | >99% | 15 |
| KRED-Ac+FDH | ECR8409 | Method 2 | 6:1 | >99% | 18 |
| KRED-Ac-V1+FDH | ECR8409 | Method 2 | 6:1 | >99% | 24 |
| KRED-Ac-V1+FDH | ECR8409 | Method 2 | 6:1 | >99% | 25 |
| KRED-Ss+GDH | LX1000HA | Method 1 | 1:10 | >99% | 9 |
| KRED-Ss+GDH | LX1000HA | Method 1 | 1:7 | >99% | 7 |
| KRED-Ss+GDH | ECR8415 | Method 1 | 1:7 | >99% | 6 |
| KRED-Ss+GDH | ECR8409 | Method 1 | 1:3 | >99% | 4 |
| KRED-Ss+GDH | LX1000HA | Method 2 | 1:5 | >99% | 14 |

### Embodiment 7: Co-immobilization of ERED and FDH/GDH

Except that the enzymes are different, the remaining steps of Methods 1 and 2 are the same as Embodiment 5.

The activity of ERED and FDH co-immobilized enzyme is detected by reactions of the following substrate 7:
3 mL of 0.1 M PB (pH 7.0-8.0) is loaded in a 10 mL reactor, and then 100 mg of the substrate 7 is added, and 10-50 mg of NAD(P)+, 80 mg of an ammonium formate and 100 mg of the co-immobilization enzyme are added. It is reacted at 30°C for 16-20 hours, and the conversion rate is tested.

The activity of ERED and GDH co-immobilized enzyme is detected by the following reaction:
3 mL of 0.1 M PB (pH 7.0-8.0) is loaded in a 10 mL reactor, and then 100 mg of the substrate 7 is added, and 10-50 mg of NAD(P)+, 120 mg of glucose and 100 mg of the co-immobilized enzyme are added. It is reacted at 30°C for 16-20 hours, and the conversion rate is tested.

Test results are shown in Table 11.

| Co-immobilized enzyme | Carrier | Method | ERED:GDH/ADH mass ratio | Conversion rate (%) | Number of cycles |
|---|---|---|---|---|---|
| ERED-Sc+FDH | LX1000HA | Method 1 | 8:1 | >99% | 6 |
| | | Method 1 | 6:1 | >99% | 6 |
| | | Method 1 | 10:1 | >99% | 7 |
| | | Method 2 | 8:1 | >99% | 17 |
| | | Method 2 | 6:1 | >99% | 15 |
| ERED-Sc+GDH | ECR8409 | Method 1 | 20:1 | >99% | 12 |
| | | Method 1 | 15:1 | >99% | 4 |
| | | Method 1 | 12:1 | >99% | 4 |
| | | Method 1 | 18:1 | >99% | 11 |
| | | Method 2 | 20:1 | >99% | 21 |
| | | Method 2 | 18:1 | >99% | 21 |
| ERED-Chr+GDH | LX1000EPN | Method 1 | 15:1 | >99% | 4 |
| | | Method 1 | 12:1 | >99% | 4 |
| | | Method 1 | 18:1 | >99% | 11 |
| | | Method 2 | 20:1 | >99% | 21 |

### Embodiment 8: Co-immobilization of IRED and FDH/GDH

Except that the enzymes are different, the remaining steps of Methods 1 and 2 are the same as Embodiment 5.

The activity of IRED and FDH co-immobilized enzyme is tested by using the following substrate 8 and according to the following method:

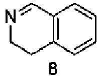

2 mL of 0.1 M PB (pH 7.0-8.0) is loaded in a 10 mL reactor, and then 100 mg of the above substrate is added, and 10-50 mg of NAD(P)+, 60 mg of an ammonium formate and 100 mg of the co-immobilization enzyme are added. It is reacted at 30°C for 16-20 hours, and the conversion rate is tested.

The activity of IRED and GDH co-immobilized enzyme is tested by the following method:
3 mL of 0.1 M PB (pH 7.0-8.0) is loaded in a 10 mL reactor, and then 100 mg of the substrate is added, and 10-50 mg of NAD(P)+, 100 mg of glucose and 100 mg of the co-immobilized enzyme are added. After it is reacted at 30°C for 16-20 hours, a conversion experiment is performed.

Test results are shown in Table 12.

| Co-immobilized | Carrier | Method | ERED:GDH/ADH mass ratio | Conversion rate (%) | Number of cycles |
|---|---|---|---|---|---|
| IRED-Str+FDH | LX1000HA | Method 1 | 4:1 | >99% | 7 |
| | | Method 1 | 6:1 | >99% | 7 |
| | | Method 1 | 8:1 | >99% | 8 |
| | | Method 2 | 4:1 | >99% | 7 |
| | | Method 2 | 6:1 | >99% | 17 |
| IRED-Bc+GDH | LX1000HA | Method 1 | 6:1 | >99% | 12 |
| | | Method 1 | 8:1 | >99% | 11 |
| | | Method 2 | 6:1 | >99% | 19 |
| | | Method 2 | 5:1 | >99% | 11 |

### Embodiment 9: Application of co-immobilized enzyme in packed bed continuous reaction

According to Method 2 in Embodiment 2, transaminase TA-Bt, coenzyme LDH and FDH are co-immobilized on a carrier LX1000HA, and the obtained co-immobilized enzyme is filled in a column-shaped reactor with a column volume of 10 mL, and the amount of the immobilized enzyme is 5.9 g.

500 g of the substrate 5, and 108 mg of an ammonium chloride are dissolved with 4.5 L of PB buffer (0.1 M, pH 8.0), pH is adjusted to 7.5-8.0 with sodium hydroxide solution, then 10-50 mg of NAD+, and 80 mg of an ammonium formate are added, and finally, the volume is fixed to 5 L with the PB buffer.

The flow rate is set to 0.1 mL/min, namely the retention time is 100 min, and a continuous reaction is performed. Effluent liquid at an outlet is detected for the conversion rate, and the conversion rate is greater than 98%. After 300 h of continuous operation, the conversion rate is not decreased. After 348 h of operation, the conversion rate is decreased by 88.4%. See Table 13 for details.

**Table 13: Reaction results of TA-Bt+LDH+FDH co-immobilized enzyme in packed bed continuous reaction**

| Enzyme | Carrier | Immobilized enzyme amount | Column volume | Retention time | Operation time | Conversion rate |
|---|---|---|---|---|---|---|
| TA-Bt+LDH+FDH | LX1000HA | 5.9g | 10mL | 100min | 300h | 98.2% |
| | | | | | 348h | 88.4% |

### Embodiment 10: Application of co-immobilized enzyme in continuous stirred tank reaction

The co-immobilized enzyme same as Embodiment 9 is used, 50 g of the co-immobilized enzyme of transaminase TA-Bt and coenzyme LDH and FDH is added to a 200 mL reactor, and 150 mL of a phosphate buffer is added.

500 g of the substrate 5, and 108 mg of an ammonium chloride are dissolved with 4.5 L of the PB buffer (0.1 M, pH 8.0), pH is adjusted to 7.5-8.0 with sodium hydroxide solution, then 10-50 mg of NAD⁺, and 80 mg of an ammonium formate are added, and finally the volume is fixed to 5 L with the PB buffer.

The substrate is continuously added to a continuous stirred tank at a rate of 0.8 mL/min (namely the retention time is 250 min), and a reaction system is extracted at an outlet at the same flow rate (a filter head is added at a tail end of a pipeline, as to prevent the immobilized enzyme from being extracted). Under this condition, the conversion rate may reach more than 92%, and the conversion rate is basically not reduced after continuous operation for 400 h. Results are shown in Table 14.

**Table 14: Reaction results of TA-Bt+LDH+FDH co-immobilized enzyme in continuous reaction of continuous stirred tank**

| Enzyme | Carrier | Immobilized enzyme amount | Reactor volume | Retention time | Operation time | Conversion rate |
|---|---|---|---|---|---|---|
| TA-Bt+LDH+FDH | LX1000HA | 50g | 200mL | 250min | 400h | >92% |
| | | | | | 412h | 86% |

### Embodiment 11: Investigation of amount of protein loaded per gram of carrier

Same as Embodiment 1, after the amino carrier is activated, the amount of a protein added per gram of the carrier is investigated, the co-immobilization of transaminase TA-Bt and coenzyme lactate dehydrogenase LDH thereof is taken as an example, the different amounts of the protein is added, as to detect a protein load amount and reaction reuse times. Results are shown in Table 15.

Same as Embodiment 4, the co-immobilization of monooxygenase CHMO-Rs and coenzymes ADH and GDH thereof is taken as an example, the different amounts of the protein is added, as to detect a protein load amount and reaction reuse times, and results are shown in Table 16. The results show that the range of the protein loaded per gram of the selected carrier is 50~200 mg, and the protein loading rate is 50%~100%.

**Table 15: Investigation of amount of protein loaded by TA-Bt and LDH mixed enzyme on different carriers**

| Carrier | Protein amount mg/g resin | Protein loading rate | Reuse times | Carrier | Protein amount mg/g resin | Protein loading rate | Reuse times |
|---|---|---|---|---|---|---|---|
| LX1000EA | 50 | 100% | 4 | ECR8309 | 80 | 99% | 7 |
| LX1000EA | 80 | 98% | 5 | ECR8309 | 100 | 92% | 9 |
| LX1000EA | 100 | 90% | 7 | ECR8309 | 120 | 80% | 9 |
| LX1000EA | 120 | 85% | 7 | ECR8409 | 80 | 98% | 14 |
| LX1000EA | 200 | 53% | 8 | ECR8409 | 100 | 95% | 15 |
| LX1000HA | 50 | 100% | 10 | ECR8409 | 120 | 90% | 15 |
| LX1000HA | 80 | 98% | 13 | ECR8315 | 80 | 99% | 10 |
| LX1000HA | 100 | 95% | 14 | ECR8315 | 100 | 94% | 12 |
| LX1000HA | 120 | 90% | 15 | ECR8315 | 120 | 90% | 13 |
| LX1000HA | 200 | 52% | 16 | ESR-1 | 80 | 99% | 8 |
| LX1000HA | 220 | 50% | 8 | ESR-1 | 100 | 92% | 9 |
| LX1000EPN | 80 | 95% | 11 | ESR-1 | 120 | 80% | 9 |
| LX1000EPN | 100 | 92% | 12 | ESR-5 | 80 | 99% | 7 |
| LX1000EPN | 120 | 86% | 11 | ESR-5 | 100 | 94% | 8 |
| HFA | 80 | 99% | 12 | ESR-5 | 120 | 81% | 9 |
| HFA | 100 | 95% | 13 | ESR-8 | 80 | 99% | 5 |
| HFA | 120 | 85% | 14 | ESR-8 | 100 | 92% | 7 |
| -/- | -/- | -/- | -/- | ESR-8 | 120 | 82% | 7 |

**Table 16: Investigation of protein loading amount of CHMO-Rs and ADH mixed enzyme on different carriers**

| Carrier | Protein amount mg/g resin | Protein loading rate | Reuse times | Carrier | Protein amount mg/g resin | Protein loading rate | Reuse times |
|---|---|---|---|---|---|---|---|
| LX1000EA | 50 | 100% | 2 | ECR8309 | 90 | 100% | 7 |
| LX1000EA | 80 | 98% | 3 | ECR8309 | 100 | 94% | 8 |
| LX1000EA | 100 | 92% | 4 | ECR8409 | 80 | 99% | 16 |
| LX1000EA | 120 | 83% | 4 | ECR8409 | 100 | 95% | 16 |
| LX1000EA | 200 | 50% | 4 | ECR8409 | 120 | 91% | 16 |
| LX1000HA | 50 | 100% | 6 | ECR8305 | 90 | 99% | 3 |
| LX1000HA | 80 | 98% | 10 | ECR8305 | 100 | 96% | 3 |
| LX1000HA | 100 | 95% | 15 | ECR8415 | 80 | 99% | 9 |
| LX1000HA | 120 | 90% | 15 | ECR8415 | 100 | 94% | 11 |
| LX1000HA | 200 | 68% | 15 | ECR8415 | 120 | 92% | 11 |
| LX1000HN | 100 | 93% | 4 | ESR-1 | 80 | 100% | 4 |
| HM100D | 100 | 90% | 3 | ESR-1 | 100 | 98% | 5 |
| LX1000EPN | 80 | 90% | 8 | ESR-1 | 120 | 90% | 5 |
| LX1000EPN | 100 | 90% | 9 | ESR-3 | 80 | 100% | 6 |
| HFA | 100 | 90% | 5 | ESR-3 | 100 | 95% | 7 |

It may be seen from data in Tables 15 and 16 that for most of the carriers, while the protein loading amount is 50-100 mg, the protein loading rate is above 90%. In the case of the same protein loading rate, the reuse times of the co-immobilized enzymes formed by the different carriers are different. It may be seen from this table that these carriers LX1000HA, LX1000EPN and ECR8409 have the better immobilized enzyme activity and stability.

### Embodiment 12: Investigation of final concentration of PEI

Same as Embodiment 2, and Method 2 (two-step method), the co-immobilized enzyme of transaminase TA-Bt and coenzyme lactate dehydrogenase LDH and ammonium formate dehydrogenase FDH thereof is prepared, after the mixed enzyme of TA-Bt and LDH is combined with the carrier, the different amounts of PEI is added, and then the second coenzyme FDH is added, to investigate the concentration range of PEI. Results are shown in Table 17.

**Table 17: Investigation of amount of PEI in two-step immobilization method of TA-Bt and coenzymes LDH and FDH thereof**

| Carrier | Final concentration of PEI | Reuse times |
|---|---|---|
| LX1000HA | 0.3% | 10 |
| LX1000HA | 0.5% | 12 |
| LX1000HA | 1% | 12 |
| LX1000HA | 3% | 12 |
| LX1000HA | 5% | 12 |
| HFA | 1% | 9 |
| HFA | 3% | 9 |
| HFA | 5% | 9 |
| HFA | 7% | 7 |
| ECR8409 | 2% | 13 |
| ECR8409 | 5% | 13 |
| ECR8409 | 7% | 13 |
| ESR-1 | 0.5% | 10 |
| ESR-1 | 2% | 11 |
| ESR-1 | 5% | 11 |

It may be seen from data in Table 17 that the effect of the concentration of PEI is basically the same in the range of 1%~5%, and beyond this range, the stability of the immobilized enzyme is decreased.

It may be seen from the above description that the above embodiments of the present invention achieve the following technical effects: the present application achieves the co-immobilization of these main enzymes and the coenzymes thereof by co-immobilizing the above main enzymes and the coenzymes thereof on the amino resin carrier, thereby it is beneficial to improve the activity of the enzyme and the recycling efficiency.

The above are only preferred embodiments of the present invention and are not intended to limit the present invention. For those skilled in the art, the present invention may have various modifications and changes. Any modifications, equivalent replacements, improvement and the like made within the spirit and principle of the present invention shall be included within a scope of protection of the present invention.

## Claims

1. A co-immobilized enzyme, wherein the co-immobilized enzyme comprises a main enzyme and a coenzyme, the number of the coenzyme is one or more, the main enzyme and the coenzyme are immobilized on an amino resin carrier, wherein the main enzyme is covalently immobilized on the amino resin carrier, and the coenzyme is covalently and/or non-covalently immobilized on the amino resin carrier,
the main enzyme is selected from the group consisting of Transaminase (TA), Amino acid dehydrogenase (AADH), Imine reductase (IRED), Ketoreductase (KRED), Enoyl reductase (ERED) and Monooxygenase (MO).

2. The co-immobilized enzyme as claimed in claim 1, wherein the transaminase is derived from *B.thuringiensis* or *Vibrio fluvialis strain JS17;*
preferably, the amino acid dehydrogenase is derived from *Bacillus cereus or from Bacillus sphaericus;*
preferably, the imine reductase is derived from *Streptomyces sp or from Bacillus cereus;*
preferably, the ketoreductase is derived from *Sporobolomyces salmonicolor* or from *Acetobacter sp.* CCTCC M209061; more preferably, the ketoreductase derived from *Acetobacter sp.* CCTCC M209061 is a mutant having the sequence of SEQ ID NO:1 or SEQ ID NO:2;
preferably, the enoyl reductase is derived from *Chryseobacterium sp. CA49 or Sewanella oneidensis MR-1;*
preferably, the monooxygenase is a cyclohexanone monooxygenase (CHMO) derived from *Rhodococcus sp. Phi1,* or from *Brachymonas petroleovorans,* or from *Rhodococcus ruber-SD1*; more preferably, the cyclohexanone monooxygenase derived from *Rhodococcus sp. Phi1* is a mutant having the sequence of SEQ ID NO:4 or SEQ ID NO:5, the cyclohexanone monooxygenase derived from *Rhodococcus ruber-SD1* is a mutant having the sequence of SEQ ID NO:7 or SEQ ID NO:8.

3. The co-immobilized enzyme as claimed in claim 1, wherein the coenzyme is selected from the group consisting of: lactate dehydrogenase, (LDH) or formate dehydrogenase (FDH) or glucose dehydrogenase (GDH) or alcohol dehydrogenase (ADH);
preferably, the Lactate dehydrogenase is a D-lactate dehydrogenase derived from *Lactobacillus helveticus*;
preferably, the formate dehydrogenase is derived from *Candida boidinii;*
preferably, the glucose dehydrogenase is glucose 1-dehydrogenase derived from *Lysinibacillus sphaericus G10*;
preferably, the alcohol dehydrogenase is derived from *Thermoanaerobium brockii;*
preferably, the co-immobilized enzyme is re-used for 4~25 cycles.

4. The co-immobilized enzyme as claimed in any one of claims 1~3, wherein the amino resin carrier is an amino resin carrier activated by glutaraldehyde;
preferably, wherein the amino resin carrier is with a C2 or C4 linker arm, more preferably, the amino resin carrier is selected from the group consisting of: SEPLITE^{®}LX1000EA, LX1000HA, LX1000NH, HFA, LX1000EPN, HM100D, Purolite^{®} Lifetech^{™} ECR8309, ECR8409, ECR8305, ECR8404, ECR8315, ECR8415, HECHENG^{®}ESR-1, ESR-3, ESR-5 and ESR-8.

5. The co-immobilized enzyme as claimed in claim 1, wherein in the co-immobilized enzyme, a mass ratio of the main enzyme and the coenzyme is 1-20:1-10;
preferably, a sum of masses of the main enzyme and the coenzyme is marked as N1, a mass of the amino resin carrier is marked as N2, N1: N2 is 50-200 mg: 1 g, and preferably, 80-120 mg :1 g.

6. The co-immobilized enzyme as claimed in claims 1 or 5, wherein
either the main enzyme and the coenzyme are covalently immobilized on the amino resin carrier; or
only the main enzyme is covalent-immobilized on the amino resin carrier and the coenzyme is non-covalently immobilized on the amino resin carrier by ionic interaction;
preferably, the coenzyme is immobilized on the amino resin carrier by PEI ionic interaction.

7. The co-immobilized enzyme as claimed in claim 6, wherein coenzyme comprises a first enzyme and a second enzyme, the main enzyme and the second enzyme are covalent-immobilized on the amino resin carrier, and the first enzyme is non-covalently immobilized on the amino resin carrier by ionic interaction.

8. The process of preparing the co-immobilized enzyme as claimed in any one of claims 1 to 7, comprising:
activating an amino resin carrier to obtain an activated carrier; and
covalently immobilizing a main enzyme on the activated carrier, and covalently and/or non-covalently immobilizing a coenzyme of the main enzyme, to obtain the co-immobilized enzyme;
wherein a number of the coenzyme of the main enzyme is one or more.

9. The process of preparing the co-immobilized enzyme as claimed in claim 8, wherein immobilizing the main enzyme and the coenzyme of the main enzyme on the activated carrier comprises:
mixing the main enzyme with the coenzyme according to the mass ratio of the main enzyme to the coenzyme as claimed in claim 5 to obtain a first mixed enzyme; followed by
immobilizing the first mixed enzyme on the activated carrier according to the ratio of N1/N2 as claimed in claim 5 to obtain the co-immobilized enzyme.

10. The process of preparing the co-immobilized enzyme as claimed in claim 8, wherein the coenzyme comprises a first enzyme, and immobilizing the main enzyme and the coenzyme of the main enzyme on the activated carrier comprises:
immobilizing the main enzyme on the activated carrier, to obtain a primary immobilized enzyme; followed by
immobilizing the first enzyme and the primary immobilized enzyme, to obtain the co-immobilized enzyme.

11. The process of preparing the co-immobilized enzyme as claimed in claim 10, wherein the coenzyme further comprises a second enzyme, and immobilizing the main enzyme and the coenzyme of the main enzyme on the activated carrier comprises:
immobilizing the main enzyme and the second enzyme on the activated carrier, to obtain the primary immobilized enzyme; followed by
immobilizing the first enzyme and the primary immobilized enzyme, to obtain the co-immobilized enzyme.

12. The process of preparing the co-immobilized enzyme as claimed in claim 10 or 11, wherein immobilizing the first enzyme and the primary immobilized enzyme by surface coating of PEI, to obtain the co-immobilized enzyme;
preferably, adding PEI to the primary immobilized enzyme until a final concentration of the PEI ranging from 0.5 w/v % to 5 w/v % to obtain a PEI-primary immobilized enzyme complex, followed by
binding the first enzyme to the PEI-primary immobilized enzyme complex, to obtain the co-immobilized enzyme.

13. The process of preparing the co-immobilized enzyme as claimed in claim 10, wherein immobilizing the first enzyme and the primary immobilized enzyme in a mass ratio of 50~150mg:1g, to obtain the co-immobilized enzyme.

14. The process of preparing the co-immobilized enzyme as claimed in claim 11, wherein a mass ratio of the main enzyme and the first enzyme is 1~20: 1~10.

15. The process of preparing the co-immobilized enzyme as claimed in claim 11, wherein a mass ratio of the main enzyme and the second enzyme is 1~20: 1~10.

16. The process of preparing the co-immobilized enzyme as claimed in claim 8, wherein the main enzyme is a transaminase, and the coenzyme has two co-factors, and the two factors are LDH and FDH, or LDH and GDH, and the process of preparing the co-immobilized enzyme comprises any one of the following:
(1) mixing the transaminase with both LDH and FDH to obtain a first enzyme mixture, and immobilizing the first enzyme mixture on the activated amino resin to obtain the co-immobilized enzyme; or
(2) mixing the transaminase with both LDH and GDH to obtain a second enzyme mixture, and immobilizing the second enzyme mixture on the activated amino resin to obtain the final co-immobilized enzyme; or
(3) mixing the transaminase with only LDH to obtain a third enzyme mixture; and immobilizing the third enzyme mixture on the activated amino resin to obtain a primary immobilized transaminase, then surface coating of PEI on the primary immobilized transaminase followed by secondary immobilization of GDH or FDH, to obtain the co-immobilized enzyme.

17. The process of preparing the co-immobilized enzyme as claimed in claim 8, wherein the main enzyme is an amino acid dehydrogenase, the coenzyme is FDH or GDH, and the process of preparing the co-immobilized enzyme comprises any one of the following:
(1) mixing the amino acid dehydrogenase with either FDH or GDH to obtain an amino acid dehydrogenase mixture; and immobilizing the amino acid dehydrogenase mixture on the activated amino resin to obtain the co-immobilized enzyme;
(2) immobilizing the amino acid dehydrogenase on the activated amino resin to obtain a primary immobilized amino acid dehydrogenase; then surface coating of PEI on the primary immobilized amino acid dehydrogenase followed by secondary immobilization of GDH or FDH to obtain the co-immobilized enzyme.

18. The process of preparing the co-immobilized enzyme as claimed in claim 8, wherein the main enzyme is an imine reductase (IRED), the coenzyme is FDH or GDH, and the process of preparing the co-immobilized enzyme comprises any one of the following:
(1) mixing the imine reductase with either FDH or GDH to obtain an imine reductase mixture; and immobilizing the imine reductase mixture on the activated amino resin to obtain the co-immobilized enzyme;
(2) immobilizing the imine reductase on the activated amino resin to obtain a primary immobilized imine reductase; then surface coating of PEI on the primary immobilized imine reductase followed by secondary immobilization of GDH or FDH to obtain the co-immobilized enzyme.

19. The process of preparing the co-immobilized enzyme as claimed in claim 8, wherein the main enzyme is a ketoreductase (KRED), the coenzyme is FDH or GDH, and the process of preparing the co-immobilized enzyme comprises any one of the following:
(1) mixing the ketoreductase with either FDH or GDH to obtain a ketoreductase mixture; and immobilizing the ketoreductase mixture on the activated amino resin to obtain the co-immobilized enzyme;
(2) immobilizing the ketoreductase on the activated amino resin to obtain a primary immobilized ketoreductase; then surface coating of PEI on the primary immobilized ketoreductase followed by secondary immobilization of GDH or FDH to obtain the co-immobilized enzyme.

20. The process of preparing the co-immobilized enzyme as claimed in claim 8, wherein the main enzyme is an enoyl reductase (ERED), the coenzyme is FDH or GDH, and the process of preparing the co-immobilized enzyme comprises any one of the following:
(1) mixing the enoyl reductase with either FDH or GDH to obtain a enoyl reductase mixture; and immobilizing the enoyl reductase mixture on the activated amino resin to obtain the co-immobilized enzyme;
(2) immobilizing the enoyl reductase on the activated amino resin to obtain a primary immobilized enoyl reductase; then surface coating of PEI on the primary immobilized enoyl reductase followed by secondary immobilization of GDH or FDH to obtain the co-immobilized enzyme.

21. The process of preparing the co-immobilized enzyme as claimed in claim 8, wherein the main enzyme is a cyclohexanone monoxygenase (CHMO), the coenzyme is FDH or GDH, and the process of preparing the co-immobilized enzyme comprise any one of the following:
(1) mixing the cyclohexanone monoxygenase with either FDH or GDH to obtain a monoxygenase mixture; and immobilizing the monoxygenase mixture on the activated amino resin to obtain the co-immobilized enzyme;
(2) immobilizing the cyclohexanone monoxygenase on the activated amino resin to obtain a primary immobilized cyclohexanone monoxygenase; then surface coating of PEI on the primary immobilized cyclohexanone monoxygenase followed by secondary immobilization of GDH or FDH to obtain the co-immobilized enzyme.

22. The process of preparing the co-immobilized enzyme as claimed in claim 8, wherein the amino resin carrier is activated by glutaraldehyde to obtain the activated carrier.

23. Use of the co-immobilized enzyme as claimed in any one of claims 1 to 7 or the co-immobilized enzyme prepared by the process of preparing the co-immobilized enzyme as claimed in any one of claims 8 to 22 in al biocatalytic process.

24. The use as claimed in claim 23, wherein the co-immobilized enzyme is used either in a batch mode or in a continuous mode,
preferably, the co-immobilized enzyme is used either in a continuous stir tank reactor (CSTR) or in a packed bed reactor (PBR);
preferably, the co-immobilized enzymet is re-used for 4 to 25 cycles in the continuous biocatalytic process.
